(19) Europäisches Patentamt
European Patent Office
Office européen des brevets

(11) Publication number: **0 000 828**
**B1**

(12) **EUROPEAN PATENT SPECIFICATION**

(45) Date of publication of patent specification: **09.03.83**

(21) Application number: **78300231.4**

(22) Date of filing: **02.08.78**

(51) Int. Cl.[3]: **C 07 D 487/04,**
A 61 K 31/40 //C07F9/65,
C07D205/08, (C07D487/04,
205/00, 209/00)

(54) **Synthetic beta-lactam compounds, a process for their preparation and compositions containing them.**

(30) Priority: **06.08.77 GB 3303777**

(43) Date of publication of application:
**21.02.79 Bulletin 79/4**

(45) Publication of the grant of the patent:
**09.03.83 Bulletin 83/10**

(84) Designated Contracting States:
**CH DE FR GB NL**

(56) References cited:
**FR - A - 2 371 449**

(73) Proprietor: **BEECHAM GROUP PLC**
**Beecham House Great West Road**
**Brentford Middlesex (GB)**

(72) Inventor: **Ponsford, Roger John**
**5, Copse Close Wimblehurst Park**
**Horsham Sussex (GB)**
Inventor: **Southgate, Robert**
**7, Tilletts Lane**
**Warnham W. Sussex (GB)**
Inventor: **Roberts, Patricia Margaret**
**52, Garlands Road**
**Redhill Surrey (GB)**

(74) Representative: **Hesketh, Alan, Dr. et al,**
**European Patent Attorney Beecham**
**Pharmaceuticals Great Burgh Yew Tree Bottom**
**Road**
**Epsom, Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Synthetic β-lactam compounds, a process for their preparation and compositions containing them

The present invention relates to β-lactam antibacterials, to a process for their preparation and to compositions containing them. French Patent Application 2371449 discloses a wide range of carbapenems obtained by synthesis, which have a carbon-bonded substituent at the C—3 position. British Patent No. 1483142 and J. Chem. Soc., Chem. Comm., 1977, 523 disclose that the compound of the formula (I).

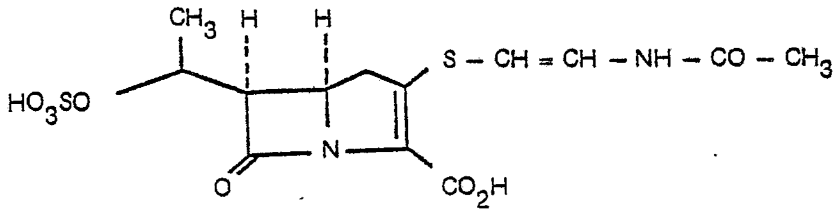

(I)

and its salts may be obtained by fermentation of strains of *Streptomyces olivaceus.* We have now found that a distinct class of synthetic antibacterial agents which contain a β-lactam ring fused to a pyrroline ring may be prepared.

Accordingly, the present invention provides the compounds of the formula (II):

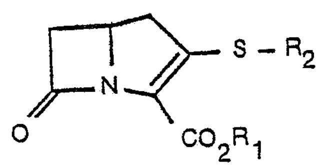

(II)

wherein:

$R_1$ is a group such that $CO_2R_1$ is a carboxylic acid group or a salt or ester thereof; and

$R_2$ is a phenyl group or a phenyl group substituted by one to four groups selected from lower alkyl, fluorine, chlorine, bromine, CN, $NO_2$, $COR_3$, $OR_3$, $SR_3$, $NH_2$, $NHCOR_3$, $NHCO_2R_3$, $CO_2R_3$ or $CO_2R_{10}$ where $R_3$ is lower alkyl, benzyl, fluorobenzyl, chlorobenzyl, bromobenzyl or nitrobenzyl and $CO_2R_{10}$ is carboxyl or a salt thereof, not more than three such groups being selected from fluorine, chlorine, bromine, CN, $NO_2$, $NH_2$, $COR_3$ or $CO_2R_3$ and not more than two such groups being selected from CN, $NO_2$ or $NH_2$.

An apt group of compounds within formula (II) includes those wherein:

$R_1$ is a group such that $CO_2R_1$ is a carboxylic acid group or a salt or ester thereof; and

$R_2$ is a phenyl group or a phenyl group substituted by one or two groups selected from lower alkyl, fluorine, chlorine, bromine, CN, $NO_2$, $COR_3$, $OR_3$, $NHCOR_3$, $NHCO_2R_3$, or $CO_2R_3$ where $R_3$ is a lower alkyl or benzyl group.

When used herein the term "lower" means that the group so described contains 1—4 carbon atoms.

A further apt group of compounds within formula (II) includes those wherein:

$R_1$ is a group such that $CO_2R_1$ is an ester group; and

$R_2$ is a phenyl group or a phenyl group substituted by one or two groups selected from fluorine, chlorine, bromine, CN, $NO_2$, $COR_3$, $OR_3$, $NHCOR_3$, $NHCO_2R_3$ or $CO_2R_3$ where $R_3$ is a lower alkyl or benzyl group.

Suitable esterifying groups $R_1$ include alkyl groups of up to 12 carbon atoms, alkenyl groups of up to 12 carbon atoms, alkynyl groups of up to 12 carbon atoms, phenyl or benzyl groups or any of the aforesaid inertly substituted by lower alkoxyl, lower acyloxyl, halogen, or nitro group. Used herein 'inertly substituted' means that the resulting group is stable and will not undergo rapid decomposition.

Particularly suitable esterifying groups $R_1$ include lower alkyl groups optionally substituted by lower alkoxyl, the benzyl group optionally substituted by lower alkoxyl, nitro, or chloro and those groups which are known to give rise to rapid in-vivo hydrolysis in penicillin esters.

Certain preferred esterifying groups $R_1$ include methyl, ethyl, methoxymethyl, 2-methoxyethyl, benzyl, methoxybenzyl and nitrobenzyl.

Other particularly preferred esterifying groups $R_1$ include those which give rise to in-vivo hydrolysable esters such as acetoxymethyl, pivaloyloxymethyl, α-ethoxycarbonyloxyethyl and phthalidyl. A preferred group $R_1$ of those giving rise to in-vivo hydrolysable esters is the phthalidyl group.

Preferred groups $R_1$ are those such that $CO_2R_1$ is a carboxylic acid salt.

The group $CO_2R_{10}$ may also be a carboxylic acid salt.

When $CO_2R_1$ is a carboxylic acid salt in compounds of the formula (II) also containing the group $CO_2R_{10}$, then $CO_2R_{10}$ is usually also a carboxylic acid salt, and normally $R_1$ and $R_{10}$ are like cations.

Typical of salts of compounds of formula (II) are conventional pharmaceutically acceptable salts such as the alkali metal and alkaline earth metal salts, in particular the sodium, potassium, calcium and magnesium salts; ammonium and substituted ammonium salts, for example the t-butylamine salt.

Particularly suitable salts are the potassium and sodium salts, especially the sodium salts.

Suitably $R_2$ is a phenyl group, optionally mono-substituted.

Suitable groups $R_2$ include the phenyl, p-chlorophenyl, m-chlorophenyl, p-nitrophenyl, m-nitrophenyl, p-ethoxycarbonylphenyl, p-fluorophenyl, p-methylphenyl, p-aminophenyl, p-acetamidophenyl, p-(4′-nitrobenzyloxycarbonylamino)phenyl and p-methoxyphenyl.

The compounds of the formula (II) are most easily provided as mixtures of those having the R- and S- configuration at C—5 i.e. the 5R and 5S forms (For example formula (III):

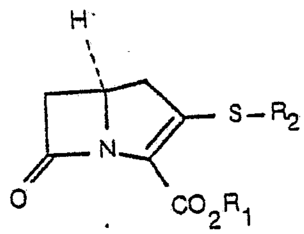

(III)

depicts the S-configuration). However this invention also provides the separate 5R and 5S forms.

A preferred sub-group of compounds within formula (II) is of formula (IV):

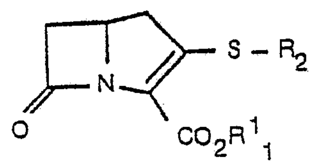

(IV)

wherein:

$R^1_1$ is a group such that $CO_2R^1_1$ is an ester group of the type which is known to undergo rapid in-vivo hydrolysis in penicillin esters; and

$R_2$ is as defined in formula (II).

Suitable groups $R^1_1$ include acetoxymethyl, pivaloyloxymethyl, $\alpha$-ethoxycarbonyloxyethyl and phthalidyl. $R^1_1$ is preferably phthalidyl.

Suitable groups $R_2$ are as so described under formula (II).

A second preferred sub-group of compounds within formula (II) is of formula (V):

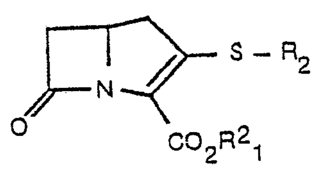

(V)

wherein:

$R^2_1$ is a group such that the compound of formula (V) is a carboxylic acid salt; and

$R_2$ is as defined in formula (II).

Particularly suitable salts are the potassium and sodium salts, especially the sodium salts.

Suitable groups $R_2$ are as so described under formula (II).

A sub-group of compounds within formula (II) of interest is of formula (VI):

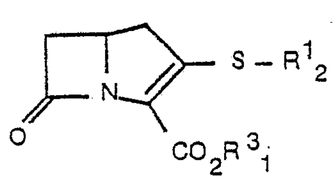

(VI)

wherein:

$R^3{}_1$ and $R^1{}_2$ are as in the Examples hereinafter.

A suitable group $R^3{}_1$ is *tert*-butyl.

Another suitable group $R^3{}_1$ is methyl.

An additional suitable group $R^3{}_1$ is benzyl.

Similarly, a suitable group $R^3{}_1$ is *p*-nitrobenzyl.

One more suitable group $R^3{}_1$ is phthalidyl.

A further suitable group $R^3{}_1$ is pivaloyloxymethyl.

Yet a further suitable group $R^3{}_1$ is sodium.

*p*-Nitrobenzyl is a preferred group $R^3{}_1$.

Phthalidyl is also a preferred group $R^3{}_1$.

Sodium is another preferred group $R^3{}_1$.

A suitable group $R^1{}_2$ is *p*-acetamidophenyl.

An additional suitable group $R^1{}_2$ is phenyl.

Similarly, a suitable group $R^1{}_2$ is *p*-nitrophenyl.

One more suitable group $R^1{}_2$ is *p*-aminophenyl.

A further suitable group $R^1{}_2$ is *p*-(4′-nitrobenzyloxycarbonylamino)phenyl.

A reaction sequence leading to the compounds of this invention is as follows:

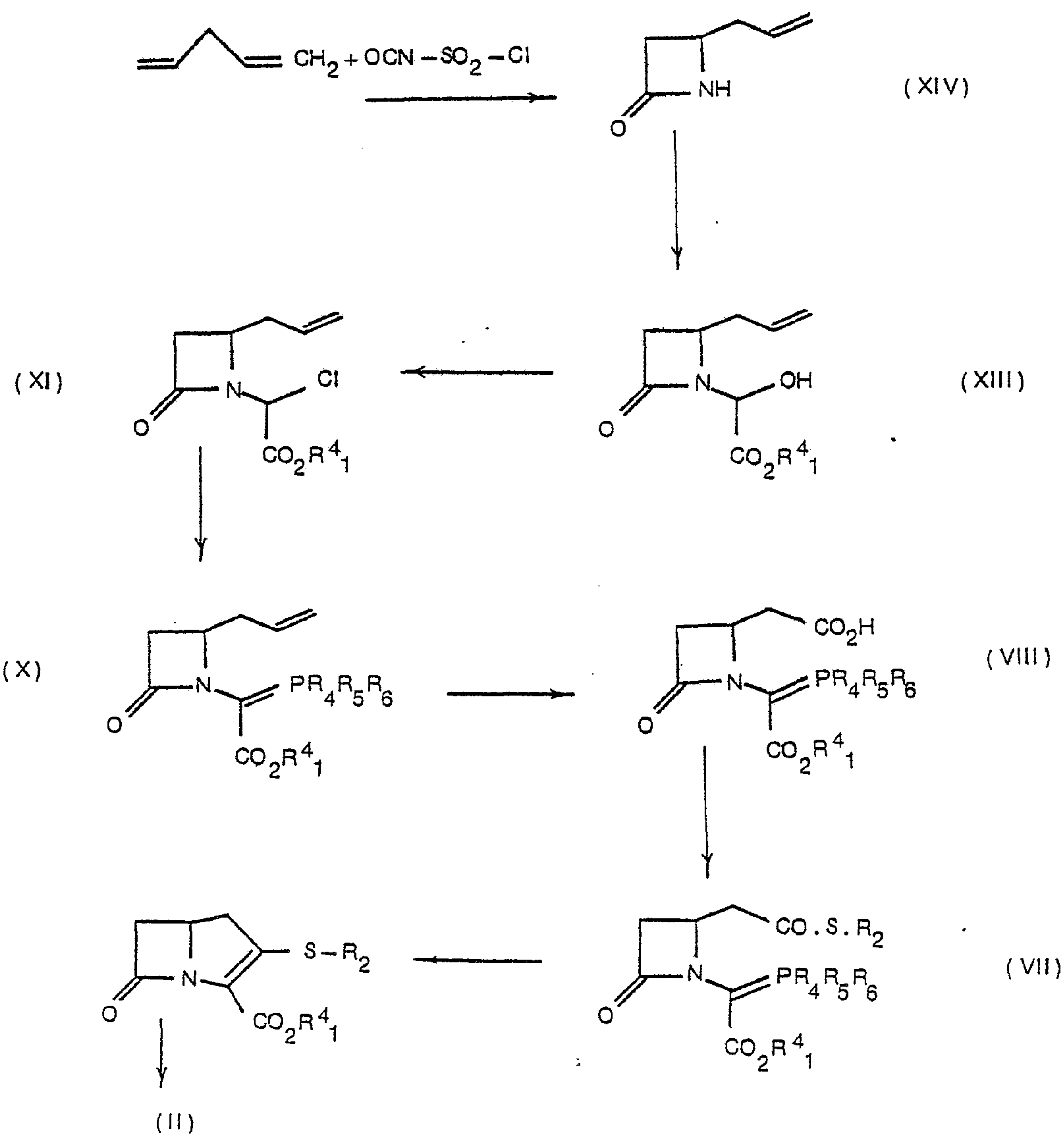

(XIV)

(XIII)

(XI)

(X)

(VIII)

(VII)

(II)

The process provided by this invention for the preparation of the compounds of the formula (II) comprises

a) the ring closing elimination of the elements of $O{=}PR_4R_5R_6$ from a compound of the formula (VII):

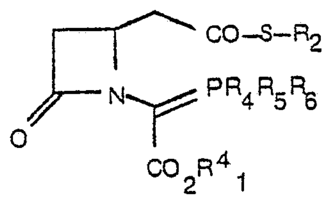

(VII)

wherein $CO_2R^4{}_1$ is an ester group, as defined in relation to formula (II) and $R_4$, $R_5$ and $R_6$ are each lower alkyl, phenyl or diloweralkylamino groups.

b) thereafter isolating the ester of the formula (II) so formed,

c) where desired de-esterifying the ester to form a free acid or its salt,

d) and thereafter optionally salifying or esterifying the free carboxylic acid so formed, or

e) thereafter optionally converting the salts so formed into a free carboxylic acid, an ester or another salt.

Most suitably $R_4$, $R_5$ and $R_6$ are each phenyl groups.

The ring closure is normally brought about by heating the compound of the formula (VII) in an inert solvent; for example temperatures of 90—120°C and more suitably 100—110°C may be employed in a solvent such as toluene or the like. The reaction is best carried out under dry conditions under an inert gas.

The ester of the compound (II) produced may be isolated by any standard method such as fractional crystallisation or chromatography. We have found that is is most convenient to separate the desired product by column chromatography.

Any convenient ester may be used in the process of this invention. Since it is frequently desirable to form a salt of compounds (II), the ester employed is preferably one which is readily converted to the parent acid or its salt by mild methods of hydrogenolysis. In a further aspect therefore the invention includes a process for preparing a salt or free acid of a compound (II) which process comprises de-esterifying an ester of a compound of formula (II). Particularly suitable esters for use in this process include benzyl esters, optionally substituted in the para position by a lower alkoxy, or nitro group or a halogen atom.

A preferred ester for use in this process is the p-nitrobenzyl ester.

Esters of compounds (II) may be de-esterified by conventional methods of hydrogenolysis.

Suitable methods include hydrogenation in the presence of a transition metal catalyst. The pressure of hydrogen used in the reaction may be low, medium or high but in general an approximately atmospheric or slightly superatmospheric pressure of hydrogen is preferred. The transition metal catalyst employed is preferably palladium on charcoal or on calcium carbonate. The hydrogenation may be effected in a suitable solvent in which the ester is soluble such as aqueous dioxan. If this hydrogenation is carried out in the presence of a base then a salt of compounds (II) is produced. Suitable bases for inclusion include $NaHCO_3$, $KHCO_3$, $Na_2CO_3$, $K_2CO_3$, $CaCO_3$, $MgCO_3$, $LiHCO_3$, $NH_4OCOCH_3$ and the like. If no base is present then hydrogenation leads to the preparation of an acid within formula (II) which may then be neutralised if desired to yield a salt. Suitable bases which may be used to neutralise acids within formula (II) include LiOH, NaOH, $NaHCO_3$, KOH, $Ca(OH)_2$ and $Ba(OH)_2$.

The salts of acids (II) may be converted to esters in conventional manner, for example by reaction with a reactive halide such as bromophthalide in solution in dimethylformamide.

The substituent group or groups within the group $R_2$ in the compounds of formula (II) may be varied by conventional reactions. Thus for example when a substituent is a nitro group it may be reduced in a conventional manner to an amino group, for example by catalysed hydrogenation. Similarly an amino group may be acylated to give a substituted amido group, for example by treatment with an acyl halide in the presence of an organic base. Substituents $NHCO_2R_3$ where $R_3$ is a benzyl group substituted as hereinbefore described may be converted to amino groups, for example by hydrogenolysis.

Compounds of the formula (II) wherein the group $R_2$ contains one or more substituents $CO_2R_{10}$ as hereinbefore defined are preferably prepared from corresponding compounds of the formula (VII) wherein $R_2$ contains corresponding substituents $CO_2R_3{}^1$ where $CO_2R_3{}^1$ is a group readily convertible to a group $CO_2R_{10}$ by hydrogenolysis. Suitable and preferred hydrogenolysis methods and esters therefor are those so described hereinbefore for the de-esterification of $CO_2R_1$ ester groups.

The compound of the formula (VII) may be prepared by the reaction of a corresponding compound of the formula (VIII):

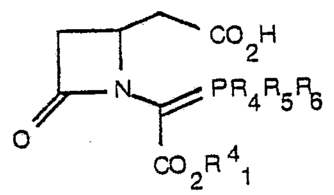

(VIII)

wherein $R_4$, $R_5$ and $R_6$ are as defined in relation to formula (VII) with a diloweralkylphosphorochloridate and a triloweralkylamine followed by reaction with a derivative of the formula (IX):

$$L^{\oplus}\ {}^{\ominus}S - R_2 \qquad (IX)$$

where $L^{\oplus}$ is a sodium or thallium (I) cation or an ammonium ion substituted by up to three organic groups, and $R_2$ is as defined in relation to formula (II).

When $L^{\oplus}$ is a substituted ammonium ion, it is preferably a tertiary ammonium ion, such as the triethylammonium ion. It is conveniently generated *in situ* by the reaction of a compound of the formula $HSR_2$ with an amine, preferably a tertiary amine.

Favourably $L^{\oplus}$ is a thallium (I) cation. Favourably $L^{\oplus}$ is a sodium cation.

A particularly suitable diloweralkylphosphorochloridate is diethylphosphorochloridate.

A particularly suitable triloweralkylamine is triethylamine.

The reaction is generally carried out in an inert organic solvent such as tetrahydrofuran at a non-extreme temperature such as 0 to 40°C, for example 15—25°C.

The compound of the formula (VIII) may be prepared by the reaction of the compound of the formula (X):

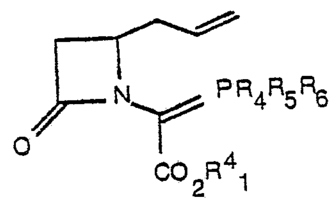

(X)

wherein $R^4{}_1$, $R_4$, $R_5$ and $R_6$ are as defined in relation to formula (VIII) with ozone in the presence of trifluoroacetic acid followed by m-chloroperbenzoic acid.

The ozonolysis is generally performed at a depressed temperature such as −40 to −80°C, for example about −70°C and in solution in an inert solvent such as methylene chloride. Excess ozone is removed by flushing with an inert gas and thereafter a solution of the peracid is added to the reaction mixture.

The compound of the formula (X) may be prepared from the corresponding compound of the formula (XI):

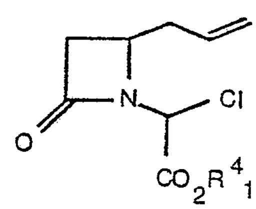

(XI)

wherein $R^4{}_1$ is as defined in relation to formula (X) with a phosphine of the formula (XII):

$$PR_4R_5R_6 \qquad (XII)$$

where $R_4$, $R_5$ and $R_6$ are as defined in relation to formula (X).

This reaction is normally effected in the presence of at least one equivalent of a base of relatively low nucleophilicity such as 2,6-lutidine at an ambient temperature in a dry solvent such as dioxan, or tetrahydrofuran.

The compound of the formula (XI) may be prepared from the corresponding carbinol of the formula (XIII):

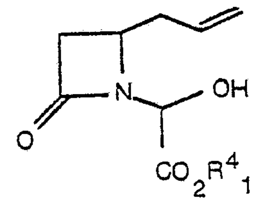

(XIII)

wherein $R^4{}_1$ is as defined in relation to formula (XI) by reaction with thionyl chloride.

This reaction is also normally effected in the presence of at least one equivalent of a base of relatively low nucleophilicity in a dry solvent such as dioxan or tetrahydrofuran but in this instance the reaction is performed at a depressed temperature, for example −30 to −10°C.

The preceding carbinol may be prepared by the reaction of a compound of the formula (XIV):

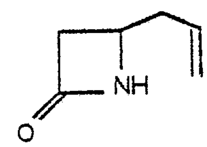

(XIV)

with a glyoxylic acid ester of the formula (XV):

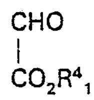

(XV)

wherein $R^4{}_1$ is as defined in relation to formula (VII).

Normally this reaction is carried out in an inert solvent at an elevated temperature, for example in dry benzene under reflux.

The compound of the formula (XIV) may be prepared as described in Description 1 hereinafter.

The present invention provides the compounds of the formulae (VII) and (VIII), as useful intermediates. The process for the preparation of these compounds also forms part of this invention.

The present invention also provides a pharmaceutical composition which comprises a compound of the formula (II) as hereinbefore defined and a pharmaceutically acceptable carrier.

The composition of the invention includes those in a form adapted for oral, topical or parenteral use and may be used for the treatment of bacterial infection in domestic animals or humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives and disintegrants in accordance with conventional pharmaceutical practice in the manner well understood by those skilled in the art of formulating antibacterial agents.

Preferably the compound of the formula (II) present in such compositions will be in-vivo hydrolysable to the parent acid or its salt.

The composition of this invention may beneficially also comprise a penicillin or cephalosporin. Certain particularly suitable penicillins for use in these compositions include amoxycillin trihydrate and sodium amoxycillin.

The present invention also provides a method of treatment and/or prophylaxis of bacterial infections in human beings or domestic animals, which method comprises the administration to the sufferer of an effective amount of a compound of the formula (II).

The following Examples illustrate this invention. The following Descriptions relate to the preparation of useful intermediates. All temperatures provided in the Examples and Descriptions are in °C.

DESCRIPTION 1

4-Allyl-1-(1′-tert-butyloxycarbonyl-1′-triphenylphosphoranylidenemethyl)azetidine-2-one

(i) *Preparation of 4-allyl azetidin-2-one*

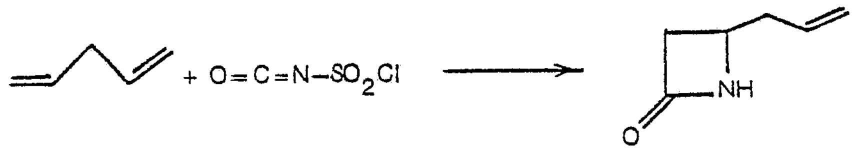

(d1) (d2) (d3)

1,4 Pentadiene(d1) (30g) and chlorosulphonyl isocyanate (d2) (35.4 ml) were mixed and allowed to stand at room temperature for 3 days, in a pressure bottle. The thick, dark syrup obtained was diluted with methylene chloride (500ml) and added dropwise to a stirred solution of sodium sulphite (66g) in water (240ml). The pH was maintained between 6.5 and 7.5 by the addition of 10% aqueous potassium hydroxide (600ml in total). The lower organic phase was separated and the aqueous phase extracted (x 2) with ethyl acetate. The combined organic extracts were dried over magnesium sulphate, filtered, and evaporated to give the crude azetidinone (d3) as a red oil (16.05g). This was sufficiently pure for use in subsequent reactions e.g. Description 1 (ii), but could be further purified by distillation b.p. 76—80°/0.2 mm Hg. $\nu_{max}$ ($CHCl_3$) 3490, 1770 (strong), 1650 (weak)$cm^{-1}$. *δppm* ($CDCl_3$) 2.39 (2H, t, J 6Hz, $CH_2$), 2.61 (1H, ddd, J 14 Hz, 2Hz, 1.5Hz, collapsing with $D_2O$ to dd, J 14Hz, 2Hz, C3—*H*), 3.10 (1H, ddd, J 14Hz, 5Hz, 2Hz, collapsing with $D_2O$ to dd, J 14Hz, 5Hz, C3—*H*), 3.55—3.91 (1H, m, C4—*H*), 4.98—6.21 (3H, complex pattern, $CH{=}CH_2$), 6.67 (1H, broad s, exch. $D_2O$) (*Found:* M, 111.0683. $C_6H_9NO$ requires M, 111.0684).

(ii) *Preparation of 4-allyl-1-(1'-hydroxy-1'-tert-butyloxycarbonylmethyl)azetidin-2-one*

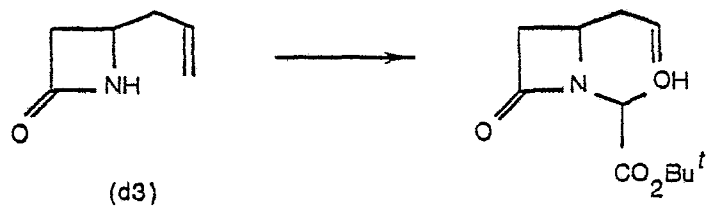

(d3) (d4)

*tert*-Butyl glyoxylate hydrate (6.22g) in benzene (120ml) was refluxed for 1 hour in a Dean-Stark apparatus to remove the water. The azetidinone (d3) (2.31g) was then added and the reaction mixture refluxed for 4 hours. Chromatography of the crude product as in Description 3(i) gave the alcohol (d4) as a pale yellow oil (4.48g). $\nu_{max}$ ($CHCH_3$) 3490, 1755, 1735, 1640 (weak)$cm^{-1}$ *δppm* ($CDCl_3$) 1.50 (9H, s, $Bu^t$), 2.20—3.25 [4H, 2.66 (1H, dd, J 3Hz, 14Hz, C3—*H*), and 3.09 (1H, dd, J 14Hz, 5Hz, C3—*H*) obscuring 2H, $CH_2$]; 3.68—4.10 (1H, m, C4—*H*), 4.47 (1H, broad s, exch. $D_2O$, *OH*); 4.98—5.37 (3H, m, sharpening with $D_2O$), 5.52—6.23 (1H, m $CH{=}CH_2$). $M^+$ at m/e 241 and (m/e +1).

(iii) *Preparation of 4-allyl-1-(1'-tert-butyloxycarbonyl-1'-triphenylphosporanylidenemethyl)azetidin-2-one*

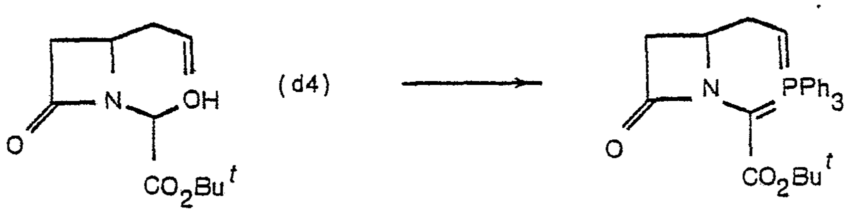

(d4) (d5)

A stirred solution of the alcohol (d4) (4.2g) in dry tetrahydrofuran (120ml) under argon, was cooled to —20°, and treated with lutidine (4.03ml) in tetrahydrofuran (15ml). Thionyl chloride (2.54ml) in tetrahydrofuran (15ml) was added dropwise. After allowing to reach 0° over 30 minutes, the solution was filtered, the lutidine hydrochloride being washed with toluene.

The combined filtrate and washings were evaporated to dryness. The residue was taken up in dry dioxan (100ml) and treated with lutidine (4.03ml) and triphenylphosphine (9.1g). After stirring at room temperature overnight, the phosphorane (d5) was isolated as in Description 3 (ii) and obtained as white crystals (4.62g) from ether mp. 188—9°, $\nu_{max}$ ($CHCl_3$) 1730, 1638, 1610$cm^{-1}$ (*Found:* C, 74.1; H, 6.8; N, 3.0, P, 6.2% $C_{30}H_{32}NO_3P$ requires C, 74.2, H, 6.6, N, 2.9, P, 6.4%).

DESCRIPTION 2

4-Allyl-1-(1'-methoxycarbonyl-1'-triphenylphosphoranylidenemethyl)azetidin-2-one

(i) *Preparation of 4-allyl-1-(1'-hydroxy-1'-methoxycarbonylmethyl)azetidin-2-one*

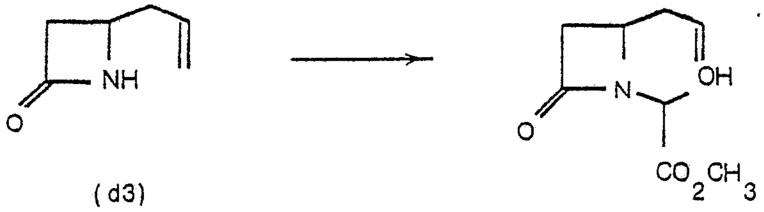

(d3)

(d6)

Methyl glyoxylate hydrate (9.75g) in benzene (500ml) was refluxed for 1 hour in a Dean-Stark apparatus to remove the water. The azetidinone (d3) (2.68g) was then added and the reaction mixture refluxed for 2 hours. A further portion of the azetidinone (1.34g) (d3) was then introduced, and refluxing continued for 3 hours. Chromatography of the crude product as in description 3 (i) gave the alcohol (d6) as a pale yellow oil (5.33g). $\nu_{max}$ ($CHCl_3$) 3500, 3350 (broad), 1760—1740 (strong), 1640 (weak)$cm^{-1}$. $\delta ppm$ ($CDCl_3$) 2.24—2.90 (3H, m, including [1H, dd, J 3Hz, 14.5 Hz at $\delta$ 2.63]), 3.11 (1H, dd, J 4.5Hz, 14.5Hz), 3.72—4.42 (5H, including [3H, s, at $\delta$ 3.90], 1H, exch. $D_2O$), 5.00—6.29 (4H, m including [1H, s, at $\delta$ 5.48]).

(ii) *Preparation of 4-allyl-1-(1'-methoxycarbonyl-1'-triphenylphosphoranylidene-methyl)azetidin-2-one*

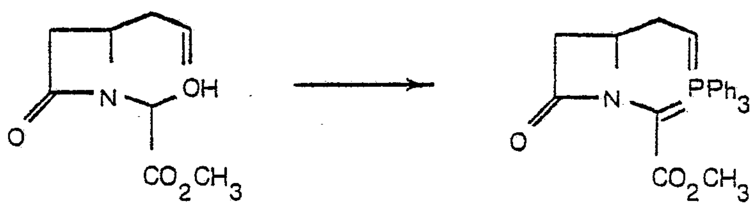

(d6) (d7)

A stirred solution of the alcohol (d6) (5.23g) in dry tetrahydrofuran (150ml) under argon, was cooled to −20°, and treated with lutidine (6.06 ml) in tetrahydrofuran (20ml). Thionyl chloride (3.83ml) in tetrahydrofuran (20ml) was added dropwise. After allowing to reach 0° over 20 minutes, the solution was filtered, the lutidine hydrochloride being washed with toluene.

The combined filtrate and washings were evaporated to dryness. The residue was taken up in dry dioxan (150ml) and treated with lutidine (6.06ml) and triphenylphosphine (13.7g). After stirring at room temperature, overnight, the phosphorane (d7) was isolated as in Description 3 (ii) and obtained as white crystals (7.3g) from ether m.p. 208—212°. $\nu_{max}$ ($CHCl_3$) 1738, 1640, 1620 $cm^{-1}$ (*Found:* C, 72.6; H, 5.9; N, 3.0%. $C_{27}H_{26}NO_3P$ requires C, 73.1; H, 5.9; N, 3.2%).

DESCRIPTION 3

(i) *4-Allyl-1-(1'-benzyloxycarbonyl-1'-triphenylphosphoranylidenemethyl)azetidin-2-one*

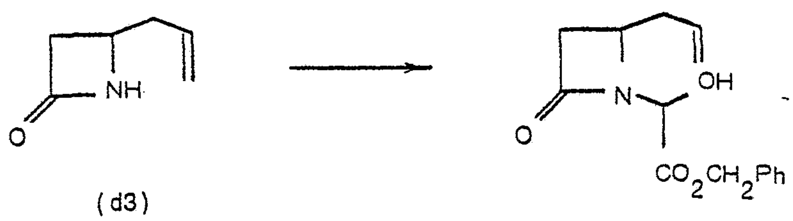

(d3)

(d8)

Benzyl glyoxylate hydrate (6g) in benzene (120ml) was refluxed for 0.5 hours in a Dean-Stark apparatus to remove the water. The azetidinone (d3) (2.13g) was added and the reaction mixture refluxed for 4 hours. The solution was cooled, evaporated, and chromatographed on silica gel, eluting with ethyl acetate-petroleum ether mixtures to give a colourless oil (5.6g) consisting mainly of the isomers of (d8) and sufficiently pure for use in subsequent reactions. Rechromatography of a small portion of this oil, eluting with chloroform gave (d8) as an oil. $\nu_{max}$ ($CHCl_3$) 3420, 1750 (strong), 1640

(weak)$cm^{-1}$. δ ppm ($CDCl_3$) 1.90—3.05 [4H, m, including δ 2.53 (1H, dd, J 15Hz, 2Hz, C3—*H*), 2.92 (1H, dd, J, 15Hz, 5Hz, C3—*H*), obscuring 2H, $CH_2$], 4.52 (1H, broad s, exch. $D_2O$, —OH), 4.85—5.90 [6H, m, including δ 5.40 (1H, broad, collapsing with $D_2O$ to singlet, *H*—C—OH) + complex pattern for $CH_2$Ph and *CH*=$CH_2$], 7.29 (5H, s).

(ii) *Preparation of 4-allyl-1-(1'-benzyloxycarbonyl-1'-triphenylphosphoranylidenemethyl) azetidin-2-one*

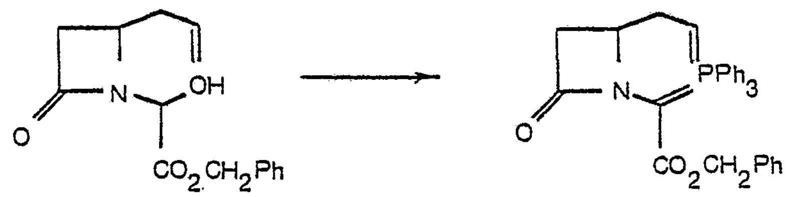

( d8 ) ( d9 )

A stirred solution of the alcohol (d8) (6.6g) in dry tetrahydrofuran (200ml), under argon, was cooled to −20°, and treated with lutidine (5.13g) in tetrahydrofuran (10ml). Thionyl chloride (5.70g) in tetrahydrofuran (20ml) was added dropwise. After allowing to reach 0°C over 20 minutes, the precipitated solid was filtered off, washing with dry toluene.

The combined filtrate and washings were evaporated to dryness and the residue taken up in dry toluene, filtered and evaporated. The gum obtained was taken up in dioxan (200ml) and treated with triphenylphosphine (12.6g) and lutidine (5.53ml). After stirring under argon at room temperature for 3 hours and standing overnight, the precipitated solid was filtered off. The filtrate was evaporated to dryness. Chromatography on silica gel eluting with ethyl acetate-petroleum ether mixtures, gave the required phosphorane, initially as a foam, which crystallized from ether (5.7g) m.p. 150—6°C. $\nu_{max}$ ($CHCl_3$) 1730, 1638, 1610 $cm^{-1}$.

## DESCRIPTION 4

4-Allyl-1-(1'-p-nitrobenzyloxycarbonyl-1'-triphenylphosphoranylidenemethyl)azetidin-2-one.

(i) *Preparation of allyl-1-(1'-hydroxy-1'-p-nitrobenzyloxycarbonylmethyl)azetidin-2-one*

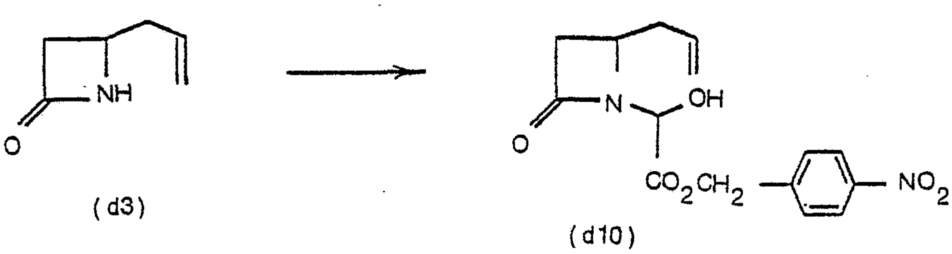

( d3 ) ( d10 )

p-Nitrobenzylglyoxylate hydrate (6.8g) in benzene (120ml) was refluxed for one hour with removal of water (Dean-Stark). The azetidinone (d3) (3g) was added and the mixture refluxed for two hours. The solution was cooled, the solvent was evaporated, and the residue was chromatographed. Elution with 80% ethyl acetate/petroleum ether (60—80°C) gàve the product. The product was rechromatographed to complete purification and collected as an oil (3.2g) (37%) $\nu_{max}$ ($CHCl_3$) 3,500 (OH), 1755 (br), 1530, 1355$cm^{-1}$. *δ ppm* ($CDCl_3$) 2.39 (2H, m $CH_2$CH=$CH_2$), 2.61 (1H, dd, J 16H, 4Hz, C3—*H*), 3.05 (1H, dd, J 16Hz, 6Hz, C3—*H*), 3.92 (1H, m, C4—*H*), 4.63 (1H, m, collapsing to a singlet on $D_2O$ exchange, *CH*—OH), 4.80 to 5.80 (6H, complex pattern including $CH_2PhNO_2$ at 5.35 OH [exchangeable] and *CH*=$CH_2$) 7.56 and 8.23 (4H, ABq, J 8Hz, aromatics).

(ii) *Preparation of 4-allyl-1-(1'-p-nitrobenzyloxycarbonyl-1'-triphenylphosphoranylidenemethyl)azetidin-2-one*

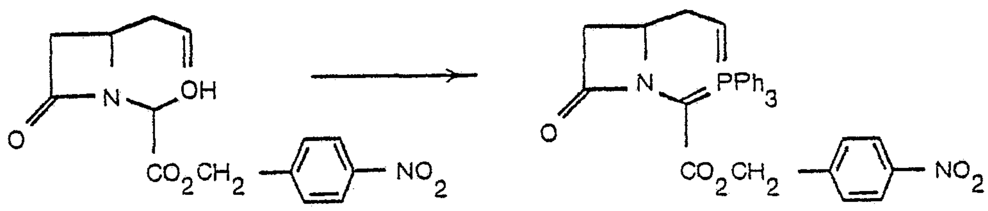

(d10) (d11)

A stirred solution of the alcohol (d10) (1.6g) in dry THF (100ml) was treated with 2,6-lutidine (1.07g) and thionyl chloride (1.19g) in THF (20ml) at −20°, and stirring was continued for 20 minutes. The mixture was filtered, the solvent was evaporated, and the residue was azeotroped twice with toluene. It was dissolved in dioxan (100ml), and 2,6-lutidine (1.07g) and triphenylphosphine (2.62g) were added. The reaction was stirred overnight at RT and filtered. The solvent was evaporated, and the residue was chromatographed. After decolourising with charcoal (ethanol/ethyl acetate) and trituration of the residue from the evaporated solution with ether, (d11) was obtained as a yellow solid (1.5g; 53%) m.p. 182—3° $\nu_{max}$ ($CHCl_3$) 1740, 1620, 1525, 1355cm$^{-1}$. (*Found:* C, 70.26; H, 5.33; N, 4.80. $C_{33}H_{29}N_2O_5P$ requires C, 70.21; H, 5.14; N, 4.96%).

DESCRIPTION 5

4-Allyl-1-(1′-pivaloyloxymethoxycarbonyl-1′-triphenylphosphoranylidenemethyl)azetidine-2-one

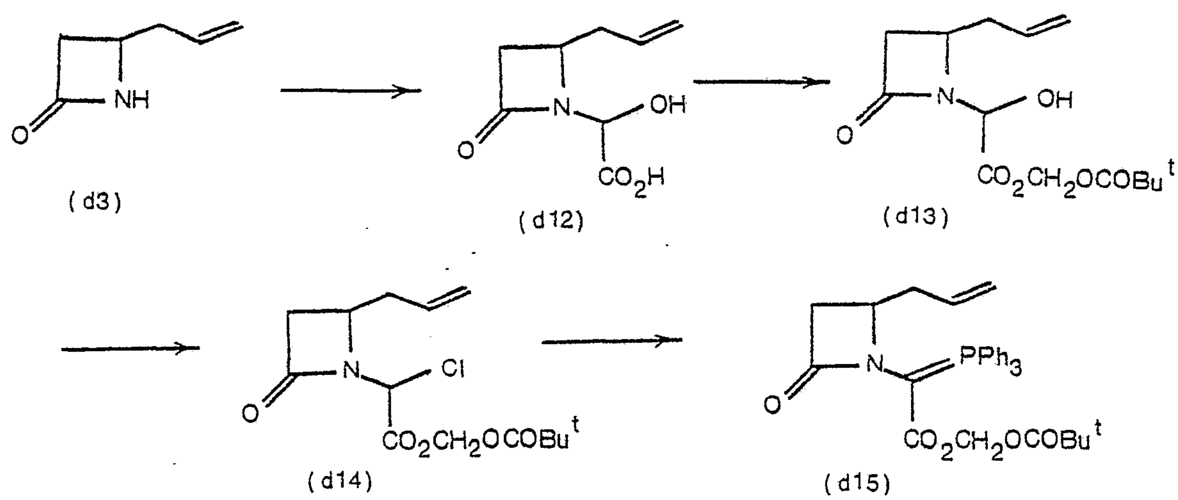

(d3) (d12) (d13)

(d14) (d15)

4-Allylazetidin-2-one(d3) (2.0 g) and glyoxylic acid monohydrate (1.75 g) were stirred together in dry dimethylformamide (10 ml) for 6 hours in the presence of 4A molecular sieves. The mixture was then cooled in an ice bath and powdered potassium carbonate (1.31 g) was added. It was allowed to warm to room temperature and stirred for 5 minutes prior to adding pivaloyloxymethyl bromide (5.3 g). The reaction was stirred overnight and then poured into a mixture of N/10 hydrochloric acid (80 ml) and ethyl acetate (80 ml). The organic phase was separated and the aqueous solution washed with further ethyl acetate (50 ml). The ethyl acetate solutions were combined, washed with saturated aqueous sodium bicarbonate, then brine and dried over sodium sulphate. It was concentrated *in vacuo* to give the crude ester (d 13) as a yellow oil (4.7 g).

The crude ester (d 13) (4.7 g) was dissolved in dry tetrahydrofuran (80 ml) and stirred at −20° under argon. It was treated with 2,6-lutidine (3.7 ml) followed over a period of 5 minutes by a solution of thionyl chloride (2.3 ml) in tetrahydrofuran (20 ml). The reaction was allowed to warm to ambient temperature over a period of $\frac{1}{2}$ hour and then filtered. The solid was washed with dry toluene and the combined filtrates concentrated under reduced pressure. The vestigial thionyl chloride was removed by two further evaporations from toluene to give the chloride (d14) as a brown oil.

The chloride (d 14) was dissolved in dry dioxane (80 ml) and treated with triphenylphosphine (8.2 g) and 2,6-lutidine (3.7 ml). The reaction mixture was stirred overnight and then filtered; the filtrate concentrated and re-dissolved in ethyl acetate (100 ml). This solution was washed free of base with N/10 hydrochloric acid (*ca* 100 ml) and then washed with brine and dried over sodium sulphate. The solution was concentrated and then chromatographed on silica gel 60 (<230 mesh) 63$\mu$m eluting with ethyl acetate/60—80° petroleum ether 7:3 to give a foam. This was dissolved in diethyl ether (20 ml) and a white solid rapidly crystallised out. This was 4-allyl-1-(1′-pivaloyloxymethoxycarbonyl-1′-tri-

phenylphosphoranylidenemethyl)azetidine-2-one (d 15) which was obtained in a yield of 3.06 g; m.p. 140—142° (ethyl acetate/60—80° petroleum ether; $\nu_{max}$ ($CHCl_3$) 2980, 1740 and 1635 $cm^{-1}$.

Example 1
t-Butyl 7-oxo-3-p-nitrophenylthio-1-azabicyclo-[3.2.0]-hept-2-ene-2-carboxylate

(a) *Preparation of 1-(1'-t-butoxycarbonyl-1'-triphenylphosphoranylidenemethyl)-4-carboxymethyl-azetidin-2-one*

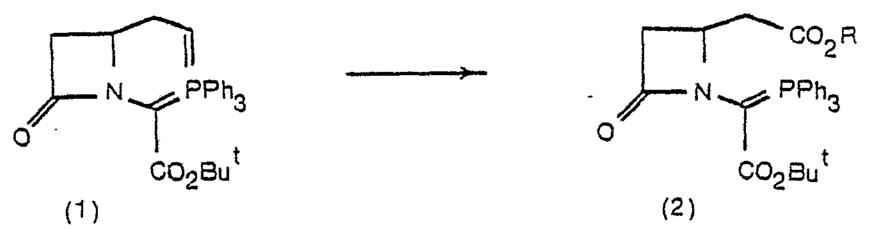

(1) (2)

(a) R=H, (b) R=$CH_2Ph$

The phosphorane (1) (Prepared as Description 1)

(2g) was dissolved in dry methylene chloride (100ml) and treated with trifluoroacetic acid (3.2ml). The solution was cooled to −70°C and ozonised until the solution turned blue. Excess ozone was removed by passing through argon and m-chloroperbenzoic acid (720 mg) in methylene chloride (20ml) was added. The mixture was allowed to reach RT and stirred overnight. The solvent was evaporated and the residue chromatographed on Merck Kieselgel 60 (<230 mesh) 63 μm. Elution with 50% ethanol/ethyl acetate gave the phosphorane-acid (2a) as a colourless foam which crystallised from ethyl acetate/ether (1.6g) as a mixture of zwitterion and trifluoroacetic acid salt, $\nu_{max}$ 1770, 1750, 1670, 1590$cm^{-1}$.

The product (1.6g) was taken up in $CHCl_3$ (50ml) and stirred with basic alumina (4g) overnight. The solution was filtered, the solvent evaporated and the residue triturated with ether to yield the phosphorane-acid (2a) as a white solid (0.9g) mp 141—3°C. $\nu_{max}$ ($CHCl_2$) 1750, 1595, 1590$cm^{-1}$.

The acid (2a) was further characterised by treatment with benzyl bromide and potassium carbonate in dimethylformamide to give the benzyl ester (2b), obtained as white crystals (ex ether) m.p. 176.5—178°C. $\nu_{max}$ ($CHCl_3$) 1735, 1640, 1610$cm^{-1}$. (*Found:* C, 72.20; H, 6.59; N, 2.28; $C_{36}H_{36}NO_5P$ requires C, 72.83; H, 6.11; N, 2,36%).

(b) *Preparation of 1-(1'-t-butoxycarbonyl-1'-triphenylphosphoranylidenemethyl)-4-(p-nitrophenyl-thiocarbonylmethyl)azetidin-2-one*

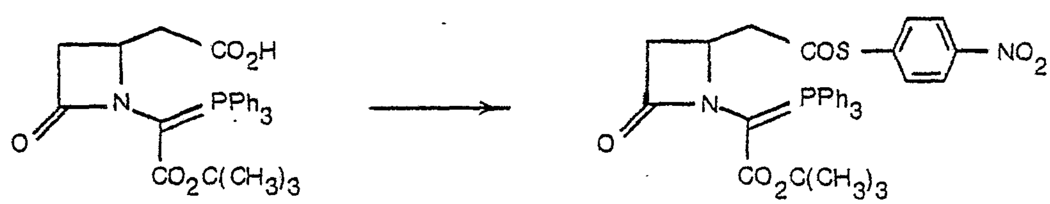

(2a) (3)

The acid (2a) (1.06g; 2mmol) and $Et_3N$ (222mg; 2.2mmole) were stirred in dry THF (20ml) at RT. A solution of diethyl phosphorochloridate (363 mg; 2.2 mmol) in THF (5ml) was added dropwise under argon at RT and the mixture stirred for 3h. The solution was filtered and to the filtrate was added (787mg; 2,2 mmol) of thallium (I) p-nitrophenylthiolate. The mixture was stirred overnight, filtered and the filtrate evaporated. Chromatography on Merck Kieselgel 60 (<230 mesh) 63 μm using ethyl acetate — petrol yielded the product (3) as an oil which crystallised from ethyl acetate/petrol as a light yellow crystalline solid (570 mg; 56% mp 115—6°C. $\nu_{max}$ ($CHCl_3$) 1740, 1720 (sh), 1640$cm^{-1}$ (*Found:* C, 65.82; H, 5.40; N, 4.32. $C_{35}H_{33}N_2O_6SP$ requires C, 65.62; H, 5.16; N, 4.38%).

(c) *Preparation of t-butyl 7-oxo-3-p-nitrophenylthio-1-azabicyclo-[3.2.0]hept-2-ene-2-carboxylate*

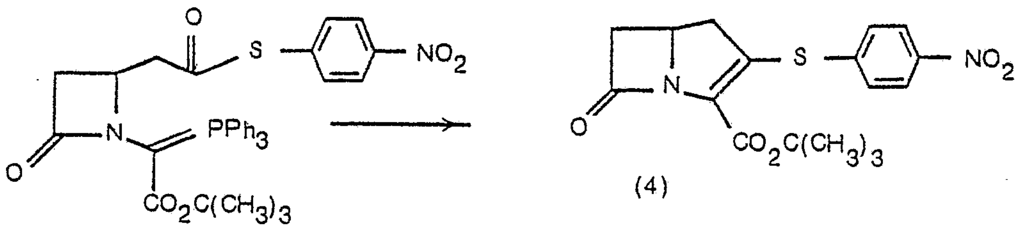

(3) (4)

The phosphorane (3) (128mg) was heated in refluxing toluene (50ml) for 15 min. The solvent was evaporated and the residue chromatographed on Merck Kieselgel 60 (<230 mesh) to yield, 15 mg of crude product. Re-chromatography yielded the title product (4) as a light yellow crystalline solid from ethyl acetate/petrol mp 148—50°C. $\nu_{max}$ ($CHCl_3$) 1790, 1710, 1695, 1525, 1345cm$^{-1}$. $\delta$ *ppm* ($CDCl_3$) 1.57 (9H, s, $CO_2C(CH_3)_3$), 2.70 (2H, d, J 9.5Hz, C4—*CH*$_2$), 2.83 (1H, dd, J 17, 3Hz, C6—*H* trans), 3.43 (1H, dd, J 17, 5Hz, C6—*H*, cis), 4.12 (1H, m, C5—*H*), 7.62 and 8.17 (4H, ABq, J 9Hz aromatic protons), $\lambda_{max}$ (EtOH) 265 nm ($\varepsilon$ = 14,300) 309 nm ($\varepsilon$ = 17,300), 346 nm (sh) ($\varepsilon$ = 13,700).

Example 2

t-Butyl 7-oxo-3-phenylthio-1-azabicyclo[3.2.0]hept-2-ene-2-carboxylate.

(a) *Preparation of 1(1'-t-butoxycarbonyl-1'-triphenylphosphoranylidenemethyl)-4-(phenylthiocarbonylmethyl)azetidin-2-one*

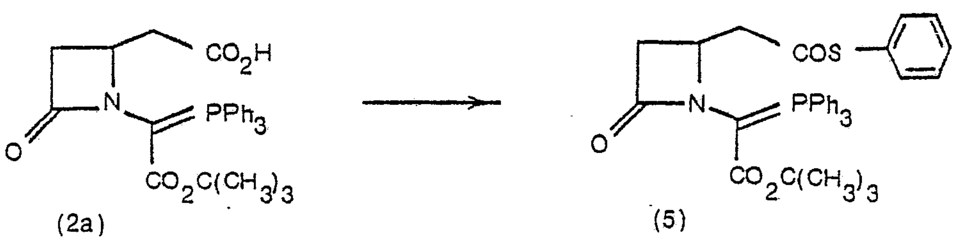

(2a) (5)

The acid (2a) (754mg; 1.5 mmol) was dissolved in dry THF (15ml) containing $Et_5N$ (167mg; 1.6 mmol) and stirred at RT. A solution of diethylphosphorochloridate (272mg; 1.6 mmol) in THF (5ml) was added dropwise to the solution under argon. Stirring was continued for 3h. The solution was filtered, and to the solution was added thallium (I) phenylthiolate (500mg; 1.6 mmol). Stirring was continued overnight. The solution was filtered, the solvent evaporated and the residue chromatographed on Merck Kieselgel 60 using ethyl acetate-petrol to yield the phosphorane (5) (700mg) as an oil. Trituration with ether gave the phosphorane as a microcrystalline solid (55mg) mp 152—5°C. $\nu_{max}$ 1740, 1700, 1640cm$^{-1}$ (*Found:* C, 70.74; H, 5.76; N, 2.43. $C_{35}H_{34}NO_4SP$ requires C, 70.59; H, 5.71; N, 2.35%).

(b) *Preparation of t butyl-7-oxo-3-phenylthio-1-azabicyclo-[3.2.0]hept-2-ene-2-carboxylate*

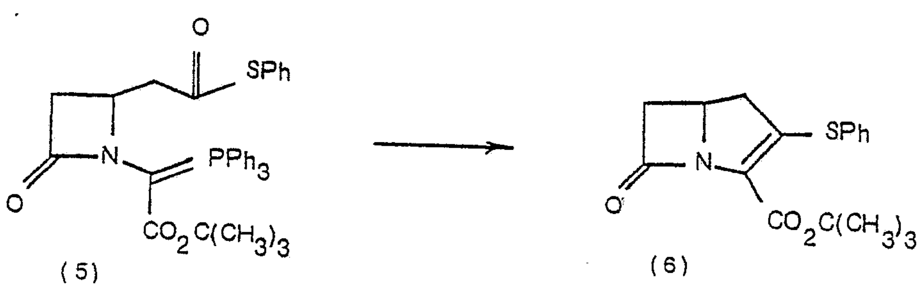

(5) (6)

The phosphorane (5) (120mg) was refluxed in dry toluene (10ml) under argon for six hours. The solvent was evaporated and the product chromatographed on Merck Kieselgel 60 to yield the title product (6) as the second compound eluted from the column (25mg; 39%). $\nu_{max}$ ($CHCl_3$) 1785, 1700cm$^{-1}$. $\delta$ *ppm* ($CDCl_3$) 1.56 (9H, s, $CO_2C(CH_3)_3$), 2.57 (2H, d, J 9Hz; C4—*CH*$_2$), 2.74 (1H, dd, J 17, 3Hz, C6—*H*, trans), 3.32 (1H, dd, J 17, 5Hz, C6—H, cis), 3.97 (1H, m. C5—*H*), 7.39 (5H, m, *Ph*). $\nu_{max}$ (EtOH) 313 nm ($\varepsilon$ = 9,510) (*Found:* M, 317.1105, $C_{17}H_{19}NO_3S$ requires 317.1085).

Example 3

Methyl 7-oxo-3-phenylthio-1-azabicyclo[3.2.0]hept-2-ene-2-carboxylate.

(a) *Preparation of 1-(1'-methoxycarbonyl-1'-triphenylphosphoranylidenemethyl)-4-carboxymethyl-azetidine-2-one*

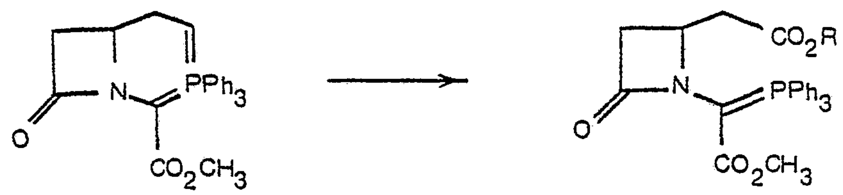

(7) (8)

a, R = H

b, R = $CH_2Ph$

The phosphorane (7) (Prepared as in Description 2) (4.47g) in dry methylene chloride (250ml), was treated with trifluoroacetic acid (7.7ml). The solution, cooled to −70°C, was ozonised until it became blue. After passing argon through to remove excess ozone, *m*-chloroperbenzoic acid (1.74g) in methylene chloride (50ml) was added. The stirred mixture was allowed to reach room temperature, and after stirring overnight, was evaporated to dryness. After re-evaporation from dry toluene the residue was chromatographed on Merck Kieselgel 60. Elution with ethyl acetate gave *m*-chlorobenzoic acid. Further elution with 10% ethanol acetate gave the phosphorane-acid (8a), partially as the trifluoroacetic acid salt, as a yellow foam (2.7g), $\nu_{max}$ ($CH_2Cl_2$) 1770, 1755, 1738, 1705—1675 (several weak peaks), 1585cm$^{-1}$.

This foam was taken up in dry methylene chloride (70ml), and stirred with basic alumina (8g), for 2 hours.

Evaporation of the filtered solution gave a foam (3g). Trituration with ether gave the zwitterionic form of the acid-phosphorane (8a), as a pale yellow solid, which was collected and dried *in vacuo* (2.35g) $\nu_{max}$ ($CH_2Cl_2$) 1750, 1740, 1590cm$^{-1}$.

The acid (8a) was characterised by treatment with benzyl bromide and potassium carbonate in dimethylformamide to give the benzyl ester (8b), as white crystals (ex ethyl acetate/petroleum ether), mp 146—8°C, $\nu_{max}$ ($CHCl_3$) 1740, 1620cm$^{-1}$ (Found: C, 71.71; H, 5.67; N, 2.44. $C_{33}H_{30}NO_5P$ requires C, 71.87; H, 5.44; N, 2.54%.

(b) *Preparation of 1-(1'-methoxycarbonyl-1'-triphenylphosphoranylidenemethyl)-4-(phenylthiocarbonylmethyl)azetidin-2-one*

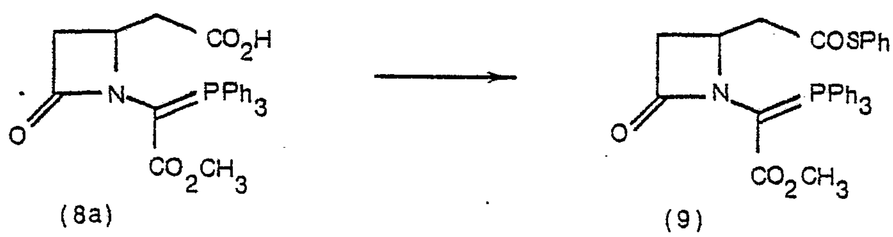

(8a) (9)

The acid (8a) (461mg; 1 mmol) was dissolved in dry THF containing $Et_3N$ (110mg; 1.1 mmol) and stirred at RT whilst a solution of diethylphosphorochloridate (152mg; 1.1 mmol) in THF (5ml) was added dropwise at RT under argon. The solution was filtered, and thallium (I) phenylthiolate (345 mg; 1.1 mmol) was added to the filtrate. Stirring was continued overnight, the solution filtered, the solvent evaporated and the residue chromatographed to yield the phosphorane (9) as an oil (400mg). Trituration with ether yielded the phosphorane as a microcrystalline solid mp 172—3°C, $\nu_{max}$ 1740, 1700, 1620cm$^{-1}$ (*Found:* C, 68.84; H, 5.24; N, 2.33. $C_{32}H_{28}NO_4SP$ requires C, 69.43; H, 5.06; N, 2.53%).

(c) *Preparation of methyl 7-oxo-3-phenylthio-1-azabicyclo[3.2.0]hept-2-ene-2-carboxylate*

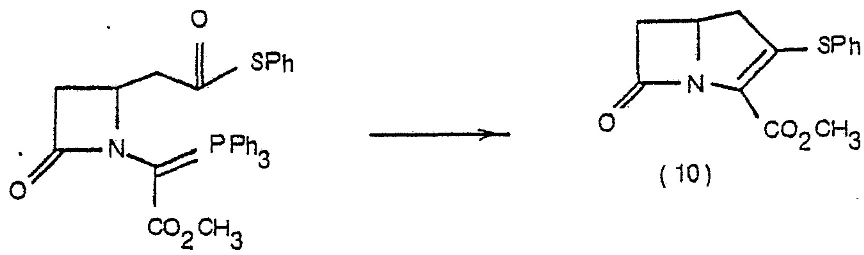

(9) (10)

The phosphorane (9) (80mg) was heated under reflux in dry toluene (5ml) for five hours. The solvent was evaporated and the product chromatographed to yield the title compound (10) (9.8mg, 25%), $\nu_{max}$ ($CHCl_3$) 1790, 1705cm$^{-1}$. $\delta$ ppm ($CDCl_3$) 2.57 (2H, d, J 9Hz; C4—$CH_2$) 2.76 (1H, dd, J 17Hz, 3Hz, C6—*H*, trans), 3.34 (1H, dd, J 17Hz, 5Hz, C6—*H*, cis), 3.82 (3H, s, $CO_2CH_3$), 4.00 (1H, m, C5—H), 7.35 (5H, m, *Ph*), $\lambda_{max}$ (EtOH) 313 nm ($\varepsilon$ = 11,400). (*Found:* M, 275.0616, $C_{14}H_{13}NO_3S$ requires 275.0616).

Example 4
Benzyl 7-oxo-3-phenylthio-1-azabicyclo[3.2.0]hept-2-ene-2-carboxylate

(a) *Preparation of 1-(1'-Benzyloxycarbonyl-1'-triphenylphosphoranylidenemethyl)-4-carboxymethyl-azetidin-2-one*

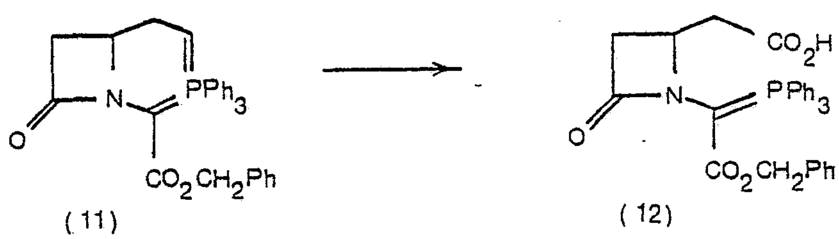

(11) (12)

The phosphorane (11, prepared as in Description 3)
(2.076g) in dry methylene chloride (120ml) was treated with trifluoroacetic acid (3.08ml). The solution, cooled to −70°C, was ozonised until it became blue. After passing argon through to remove excess ozone, *m*-chloroperbenzoic acid (0.69g) in methylene chloride (25ml) was added. The stirred mixture was allowed to reach room temperature. After stirring for 3 days, work up and chromatography as in Example 1, gave the phosphorane acid (12) partially as the trifluoroacetic acid salt as a yellow foam (1.215g), $\nu_{max}$ ($CHCl_3$) 1770 (shoulder) 1750, 1730, 1700, 1665, 1590, 1575cm$^{-1}$.

This foam was taken up in chloroform (20ml) and stirred with basic alumina (4g) for 4 hours. Evaporation of the filtered solution gave the zwitterionic form of the acid-phosphorane (12) as a foam (0.855g) $\nu_{max}$ ($CHCl_3$) 1735, 1590, 1585, 1575cm$^{-1}$.

(b) *Preparation of 1(1'-benzyloxycarbonyl-1'-triphenylphosphoranylidenemethyl)-4-(phenylthiocarbonylmethyl)azetidin-2-one.*

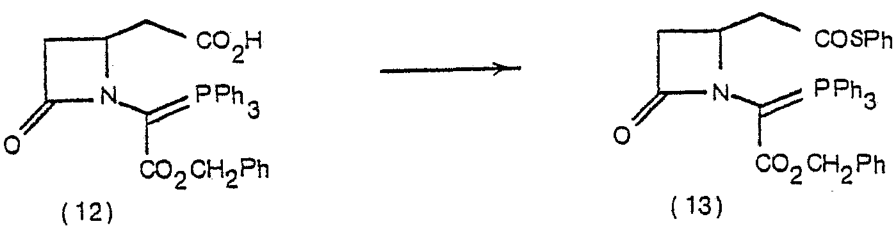

(12) (13)

The acid (12) (1.07g; 2 mmol) and $Et_3N$ (220mg; 2.2 mmol) in dry THF (30ml) were stirred at RT and a solution of diethyl phosphorochloridate (380mg; 2.2 mmol) in THF (5ml) was added dropwise under argon and stirred for three hours at RT. To the solution was added thallium (I) phenylthiolate (686mg; 2.2 Mmol) and the mixture stirred overnight. The solution was filtered and the solvent

evaporated to yield an oil. Chromatography on Merck Kieselgel 60 using ethyl acetate/petrol by gradient elution gave the title product (13), which crystallized from ethyl acetate/ether as a microcrystalline solid mp 160—1°C, $\nu_{max}$ 1745, 1705, 1620cm$^{-1}$ (*Found:* C, 72.12; H, 5.28,; N, 2.15. $C_{38}H_{32}NO_4SP$ requires C, 72.50; H, 5.09; N, 2.23%).

(c) *Preparation of benzyl 7-oxo-3-phenylthio-1-azabicyclo[3.2.0]hept-2-ene-2-carboxylate*

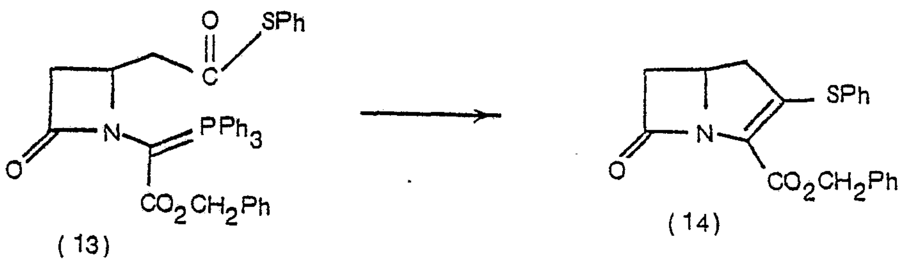

(13) (14)

The phosphorane (13) (150mg) was refluxed in dry toluene (100ml) under argon for nine hours. The solvent was evaporated and the product chromatographed on Merck Kieselgel 60 using ethyl acetate/petrol as eluant to yield the title compound (14) as the major product (25 mg; 30%), $\nu_{max}$ ($CHCl_3$) 1790, 1705 cm$^{-1}$. $\delta$ *ppm* ($(CD_3)_2CO$) 2.72 (2H, s, C4—$CH_2$), 2.87 (1H, dd, J 17, 3Hz, C6—*H*, trans), 3.32 (1H, dd, J 17, 5½Hz, C6—*H*, cis), 4.05 (1H, m, C5—*H*), 5.23 (2H, s, $CO_2CH_2Ph$), 7.20—7.70 (10H, m, S*Ph* and $CO_2CH_2Ph$), $\lambda_{max}$ (EtOH) 317 nm ($\varepsilon$ = 12,250). (*Found:* M, 351.0929, $C_{20}H_{17}NO_3S$ requires 351.0929).

Example 5

p-Nitrobenzyl 7-oxo-3-phenylthio-1-azabicyclo[3.2.0]hept-2-ene-2-carboxylate

(a) *Preparation of 1-(1'-p-Nitrobenzyloxycarbonyl-1'-triphenylphosphoranylidenemethyl)-4-carboxymethylazetidin-2-one*

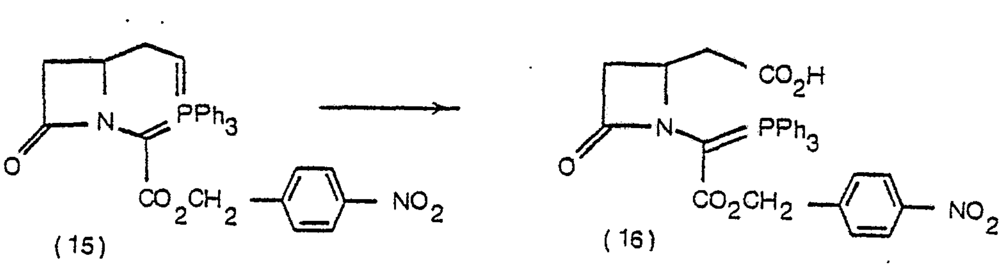

(15) (16)

The phosphorane (15, prepared as in Description 4) (2.82g) in dry methylene chloride (125ml) was treated with trifluoroacetic acid (4ml) at 0°. The solution was cooled to −70° and treated with ozone until blue. Argon was passed through to remove excess ozone, and m-chloroperbenzoic acid (0.9g) in methylene chloride (20ml) was added, and the mixture was stirred at RT overnight. The solvent was evaporated, and the resulting white solid was dissolved in ethyl acetate and chromatographed on silica gel. Elution with 10% ethanol/ethyl acetate gave the product as the trifluoroacetic acid salt. The product was stirred in ethyl acetate with basic alumina (6g) for two hours. Evaporation of the solvent and trituration of the residue with diethyl ether gave the acid (16) as a light yellow hygroscopic solid (2g; 69%). A small portion crystallised from diethyl ether gave a microcrystalline solid m.p. 127—33°C. $\nu_{max}$ ($CHCl_3$) 1745, 1600, 1355, 1115cm$^{-1}$. (*Found:* C, 64.59; H, 4.82; N, 4.66. $C_{32}H_{27}N_2O_7P$. ½$H_2O$ requires C, 64.97; H, 4.73; N, 4.73).

(b) *Preparation of 1-(1'-p-nitrobenzyloxycarbonyl-1'-triphenylphosphoranylidenemethyl)-4-(phenylthiocarbonyl)azetidin-2-one*

(i)

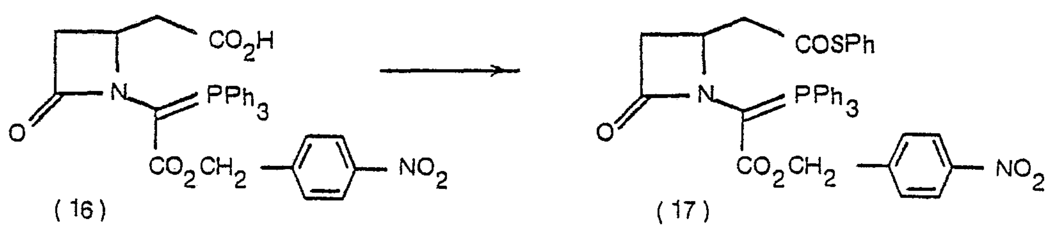

(16) (17)

The acid (16) (1.16g) and $Et_3N$ (220mg) in dry THF (30ml) were stirred at RT, and a solution of diethyl phosphorochloridate (380mg) in THF (5ml) was added dropwise under argon and stirred at RT for three hours. To the solution was added thallium (I) phenylthiolate (686mg), and the mixture was stirred overnight. The solution was filtered, and the solvent was evaporated. Chromatography yielded the phosphorane thioester (17) as a light tan solid from diethyl ether/ethyl acetate (950mg; 70%). Recrystallisation from ethyl acetate gave a white solid m.p. 186—8°C. $\nu_{max}$ ($CHCl_3$) 1745, 1705, 1615, 1350, 1145cm$^{-1}$. (*Found:* C, 67.40; H, 4.68; N, 4.02. $C_{38}H_{31}N_2O_6PS$ requires C, 67.66; H, 4.60; N, 4.15%).

(ii) Alternative Procedure

The acid, (16, prepared as in (a) above) (2.90g) and $Et_3N$ (550mg) in dry THF (75ml) were stirred at RT and a solution of diethyl phosphorochloridate (950mg) in THF (10ml) was added dropwise under argon and stirred for three hours at RT. Thiophenol (550mg) was added to the solution followed by $Et_3N$ (550mg) and the mixture stirred at RT. for two hours. The solvent was evaporated and the residue chromatographed to yield the thioester-phosphorane as a light tan solid from ethyl acetate/ether. Recrystallisation from ethyl acetate gave a white solid, shown by m.p., i.r. and analysis to be identical with (17) as in b (i) above.

(c) *Preparation of p-nitrobenzyl 7-oxo-3-phenylthio-1-azabicyclo[3.2.0]hept-2-ene-2-carboxylate*

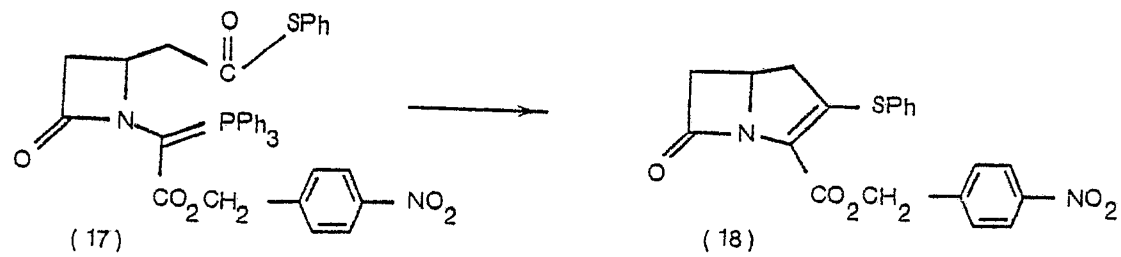

(17) (18)

The phosphorane (17) (550mg) was refluxed in dry toluene (500ml) for 24 hours with removal of water (Dean-Stark) under argon. The solvent was evaporated and the product chromatographed on florisil (200—300 U.S. mesh) using ethyl acetate/petrol (60—80°) as eluant to yield the title compound (52mg; 18%) as a crystalline solid from benzene/petrol (60—80°C) mp 112—4°C. $\nu_{max}$ ($CHCl_3$) 1790, 1705 cm$^{-1}$. $\delta$ ppm ($CDCl_3$) 2.63 (2H, d, J 9Hz, C4—$CH_2$), 2.81 (1H, dd, J17, 3Hz, C6—*H* trans), 3.38 (1H, dd, J17, 5½Hz, C6—*H*, cis), 4.04 (1H, m, C5—*H*), 5.26 and 5.49 (2H,ABq J14Hz, benzylic $CH_2$), 7.36 (5H, m, S*Ph*), 7.60 (d, 2H, J9Hz, *Ph*$NO_2$), 8.15 (d, 2H, J9Hz, *Ph*$NO_2$) $\lambda_{max}$ (EtOH) 266 nm ($\varepsilon$ 10,200), 317 nm ($\varepsilon$ 8,900). (*Found:* C, 60.56; H, 3.93; N, 6.91% $C_{20}H_{16}N_2O_5S$ requires C, 60.61; H, 4.04; N, 7.07%).

Example 6

Benzyl 7-oxo-3-p-acetamidophenylthio-1-azabicyclo[3.2.0]hept-2-ene-2-carboxylate

(a) *Preparation of 4-(p-Acetamidophenylthiocarbonylmethyl)-1(1'-benzyloxycarbonyl-1'-triphenylphosphoranylidenemethyl)azetidin-2-one*

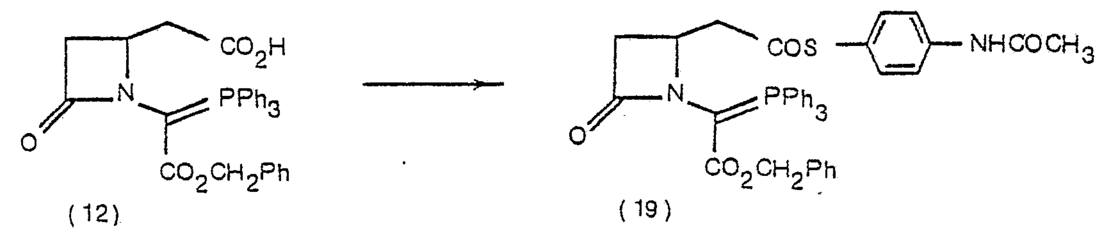

(12) (19)

The acid (12; prepared as in Example 4a) (268mg) in dry tetrahydrofuran (10ml) under argon was treated with triethylamine (76mg), followed by diethylphosphorochloridate (130mg), diluted with tetrahydrofuran (5ml). After stirring at room temperature for 3 hours, the solution was added to freshly prepared sodium *p*-acetamidophenylthiolate in tetrahydrofuran [prepared by treating p-acetamidophenylthiol (92mg) in dry tetrahydrofuran (5ml)/hexamethylphosphoramide (89mg), under argon, at 0°, with sodium hydride (26mg of a 50% suspension in oil)].

The stirred mixture was allowed to warm up from 0° to room temperature over 30 minutes, and

after a further hour was diluted with ethyl acetate (60ml), washed with water, then brine, dried over magnesium sulphate and filtered and the filtrate was evaporated. Chromatography on Merck Kieselgel 60 (10g), eluting with ethyl acetate, gave the required thioester (19) as white crystals (128mg). Recrystallisation from chloroform/ethyl acetate gave white needles m.p. 181—3°C, $\nu_{max}$ (Nujol) 1730, 1700, (broad), 1620, 1590cm$^{-1}$. $\nu_{max}$ (KBr) 1730, 1695 (broad), 1620, 1595cm$^{-1}$. (Found: C, 69.81; H, 5.32; N, 4.35. $C_{40}H_{35}N_2SO_5P$ requires C, 70.00; H, 5.10; N, 4.08%).

*Note:* This thioester (19) was also prepared substituting the sodium thiolate mixture by solid thallium (I)*p*-acetamidophenylthiolate. A comparable yield was obtained.

(b) *Preparation of Benzyl 7-oxo-3-p-acetamidophenylthio-1-azabicyclo[3.2.0]hept-2-ene-2-carboxylate*

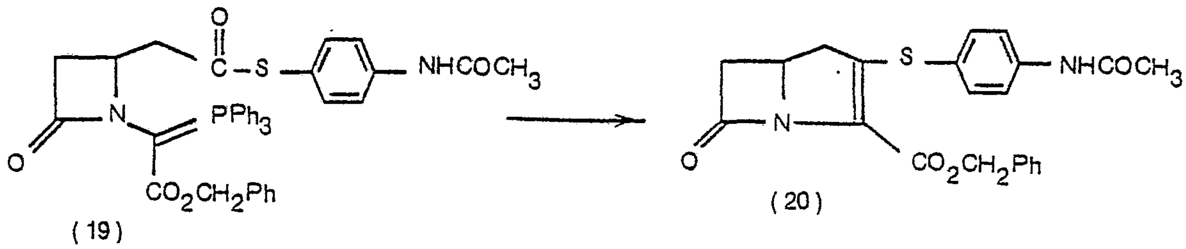

(19) (20)

The phosphorane (19) (136mg) was suspended in dry toluene (10ml) and the mixture was evaporated to dryness. The residue was suspended in dry toluene (60ml), and the mixture was degassed and heated, under argon, under reflux with a Dean-Stark head. After 5 hours the pale yellow solution was cooled to room temperature, and the solvent was evaporated. The residue was taken up in ethyl acetate (12ml), and left overnight at 5°, when buff coloured crystals were obtained. These were collected (97.5mg) and shown by thin layer chromatography to be recovered starting material. The solution was evaporated, and the residue was chromatographed on Merck Keiselgel 60, eluting with ethyl acetate/petrol mixtures to give the required compound (20), initially as a gum (7.5mg) contaminated with triphenylphosphine oxide. Trituration of this gum with diethyl ether gave (20), as a white solid (4.2mg), $\nu_{max}$ ($CHCl_3$) 1782, 1700 (shoulder), 1695, 1590cm$^{-1}$. $\lambda_{max}$ (ethanol) 312nm and 245 nm. $\delta$ *ppm* ($CDCl_3$) 2.12 (s, 3H, $CH_3$); 2.57 (2H, d, J 8Hz, C4—$CH_2$), 2.74 (1H, dd, J 16Hz, 3Hz, C6—*H*); 3.32 (1H, dd, J 16Hz, 5Hz, C6—*H*); 4.00 (1H, m, C5—*H*); 5.27 (2H, s, $CH_2$Ph); 7.12—7.62 (14H, *Ar* + $Ph_3P$=O); 7.71 (1H, s, N*H*).

Example 7

Benzyl 7-oxo-3-p-aminophenylthio-1-azabicyclo[3.2.0]hept-2-ene-2-carboxylate

(a) *Preparation of 4-(p-Aminophenylthiocarbonylmethyl)-1(1'-benzyloxycarbonyl-1'-triphenylphosphoranylidenemethyl)azetidin-2-one*

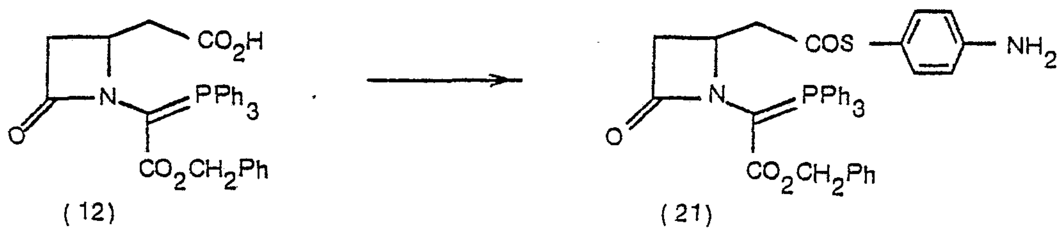

(12) (21)

The acid (12, prepared as in Example 4a) (1.675g) in dry tetrahydrofuran (80ml), under argon, was treated with triethylamine (0.472g) and diethylphosphorochloridate (0.807g). After stirring at room temperature for 3 hours the reaction mixture was added to freshly prepared sodium p-aminophenylthiolate in tetrahydrofuran [prepared by treating *p*-aminophenylthiol (0.428g) in dry tetrahydrofuran (30ml)/hexamethylphosphoramide (0.505g), under argon at 0°, with sodium hydride (0.165g of a 50% suspension in oil)].

The stirred mixture as allowed to warm from 0° to room temperature. After a total of 1.5 hours, work-up as in Example 6, followed by chromatography on Merck Keisel gel.60 (60g), eluting with from 50% ethyl acetate in petroleum ether (60—80°C) to neat ethyl acetate gave the required thioester (21) as a cream coloured solid (0.9g). Recrystallisation of a portion from hot ethyl acetate/petroleum ether (60—80°) gave "spherical crystals" m.p. 114.5—118°, $\nu_{max}$ ($CHCl_3$) 3330, 1738, 1685 (broad, weak), 1620, 1600 (shoulder)cm$^{-1}$.

(b) *Preparation of Benzyl 7-oxo-3-p-aminophenylthio-1-azabicyclo[3.2.0]hept-2-ene-2-carboxylate*

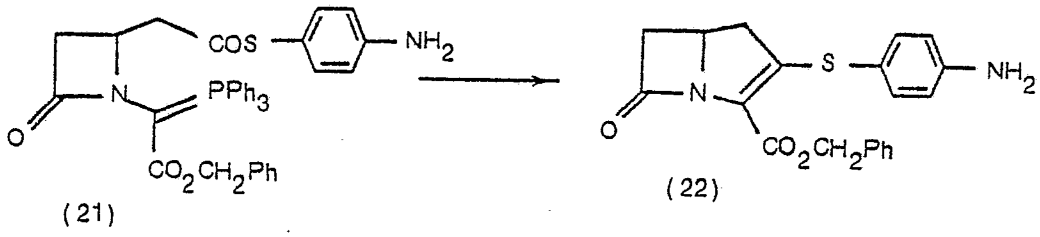

(21) (22)

The phosphorane (21) (0.461g) was taken up in dry toluene, and the mixture was evaporated to dryness. The residue was suspended in dry toluene (250ml), and the mixture was degassed and heated to reflux. A pale yellow solution was obtained. After refluxing for 6.5 hours the solution was left in the refrigerator overnight. The toluene solution was decanted from some gummy material and evaporated to dryness. Trituration of the residue with dry diethyl ether gave recovered (21) as a solid (414mg). The ethereal solution was evaporated to dryness and the residue was taken up in toluene and chromatographed on Merck Keiselgel 60 (4g), eluting with mixtures of ethyl acetate and petroleum ether (60—80°C), to give the required bicyclic product (22) as a gum (3.5mg). $\nu_{max}$ ($CHCl_3$) 3300 (weak), 1780 (strong), 1700, 1685, (weak), 1620, 1600cm$^{-1}$. $\lambda_{max}$ (ethanol) at 314 nm and 261.5 nm.

(Treatment of this p-aminophenylthio compound (22) with triethylamine/acetyl chloride gave the *p*-acetamidophenylthio compound (20) as shown by thin layer chromatography).

Example 8

Preparation of Benzyl 7-oxo-3-p-aminophenylthio-1-azabicyclo[3.2.0]hept-2-ene-2-carboxylate (Alternative Procedure)

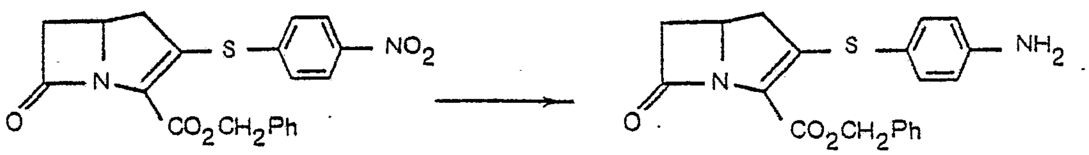

(26) (22)

A catalyst of 10% palladium on charcoal (Engelhard 4505, 50mg) suspended in 90% aqueous ethanol (10ml) was prehydrogenated for 20 minutes at room temperature/atmospheric pressure. Benzyl 7-oxo-3-*p*-nitrophenylthio-1-azabicyclo[3.2.0]hept-2-ene-2-carboxylate (50mg) partially dissolved in ethanol (10ml) was added, and the mixture was hydrogenated at room temperature and atmospheric pressure for 1.5 hours. The catalyst was removed by filtration through "High-Flo", and the colourless filtrate was evaporated to give an oil (35mg) identical by TLC, u.v. and I.R. to the product (22) obtained in Example 7.

Example 9

Benzyl 7-oxo-3-(4-p-nitrobenzyloxycarbonylaminophenylthio)-1-azabicyclo[3.2.0]hept-2-ene-2-carboxylate

(a) *Preparation of 1-(1'-Benzyloxycarbonyl-1-triphenylphosphoranylidenemethyl)-4-(p-nitrobenzyloxycarbonylaminophenylthiocarbonylmethyl)azetidin-2-one*

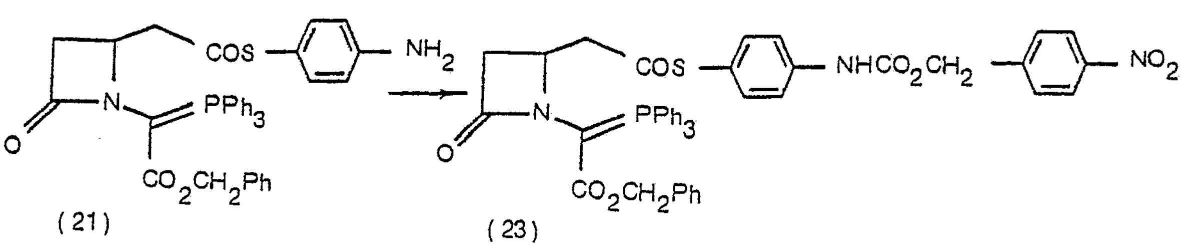

(21) (23)

The phosphorane (21) (438mg) in dry tetrahydrofuran (60ml), under argon was treated with powdered sodium hydroxide (27.2mg) and *p*-nitrobenzylchloroformate (147 mg). After stirring at room

temperature for 1.5 hours, the solvent was removed by evaporation, and the residue was taken up in chloroform, washed with brine, dried over magnesium sulphate and filtered, and the filtrate was evaporated. Chromatography on Merck Keiselgel 60, eluting with from 50% ethyl acetate in petroleum ether (60—80°) to neat ethyl acetate gave the required thioester (23) as a white solid (255mg), contaminated with a little starting material (22). Further elution with a mixture of chloroform/ethyl acetate gave a further (109mg) of the required product (23). A portion was recrystallised from chloroform/ether. The crystals were dried *in vacuo* at 100°C for a total of 11 hours, to give fine white crystals m.p. 205—7°C (dec.) $\nu_{max}$ (nujol) 1742, 1738, 1700, 1680 (weak), 1605, 1590, 1518, 1345cm$^{-1}$. (*Found:* C, 65.52; H, 4.46; N, 4.86. $C_{46}H_{38}N_3O_8SP.H_2O$ requires:— C, 65.50; H, 4.75; N, 4.98%).

(b) *Preparation of Benzyl 7-oxo-3-(4-p-nitrobenzyloxycarbonylaminophenylthio)-1-azabicyclo[3.2.0]-hept-2-ene-2-carboxylate*

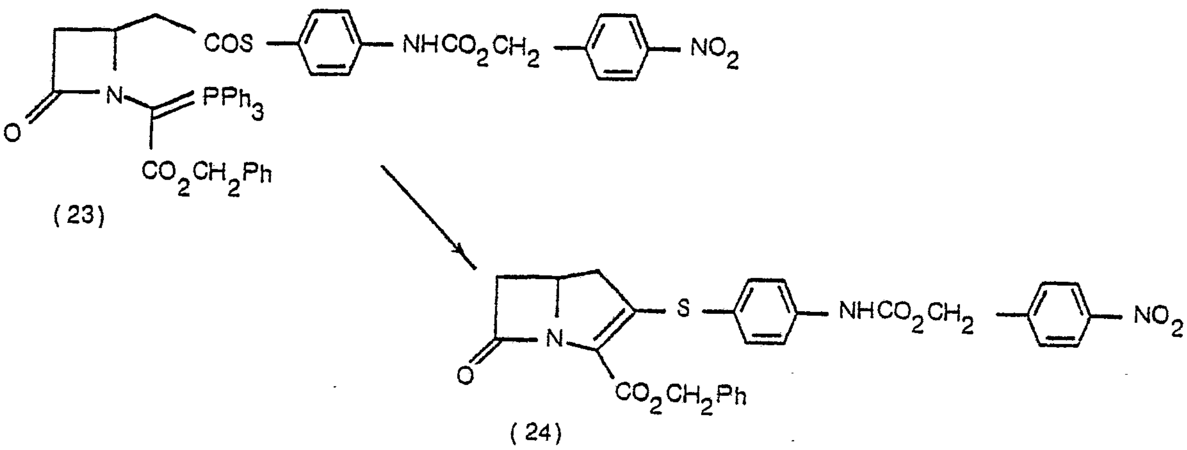

(23)

(24)

The phosphorane (23) (850mg) was suspended in dry toluene (500ml) and heated under an atmosphere of argon under reflux using a Dean-Stark apparatus to remove water. A clear, pale yellow solution was obtained. After refluxing for 6.5 hours the slightly darker solution was cooled and reduced by evaporation to about 150ml. On storing overnight at 5°, recovered starting material (551mg) was precipitated. The solution was evaporated, and the residue was chromatographed on florisil (200—300 U.S. mesh). Elution with 20—30% ethyl acetate in petroleum ether (60—80°C) gave the required bicyclic compound (24) contaminated with triphenylphosphine oxide and some non-bicyclic $\beta$-lactam containing material as a gum (37.5mg). Trituration of this gum with diethyl ether gave a white solid (14.8mg). This solid was recrystallised from ethyl acetate/petroleum ether (60—80°C) to give the bicyclic compound (24) as fine white crystals m.p. 130—8°C, $\nu_{max}$ ($CHCl_3$) 1780 (strong) 1738, 1700, 1685, 1510, 1345cm$^{-1}$. $\lambda_{max}$ (ethanol) 251 nm ($\varepsilon = 23{,}700$) and 316 nm ($\varepsilon = 16{,}700$). $\delta$ *ppm* ($CDCl_3$) 2.56 (2H, d, J 9 Hz, C4—*H*'s) 2.74 (1H, dd, J 16Hz, 2.5 Hz, C6—*H*); 3.35 (1H, dd, J 16Hz, 5Hz, C6—*H*); 3.85 (1H, centre of m, C5—*H*); 5.24 (2H, s, C*H*$_2$Ar); 5.26 (2H, s, C*H*$_2$Ar); 6.76 (1H, s, N*H*); 7.15—7.55 (11H, *Ar*); 8.0—8.25 (2H, *Ar*).

## Example 10

### Benzyl 3-p-nitrophenylthio-7-oxo-1-azabicyclo[3.2.0]hept-2-ene-2-carboxylate

(a) *Preparation of 1-(1'-Benzyloxycarbonyl-1'-triphenylphosphoranylidenemethyl)-4-(p-nitrophenylthiocarbonylmethyl)azetidin-2-one*

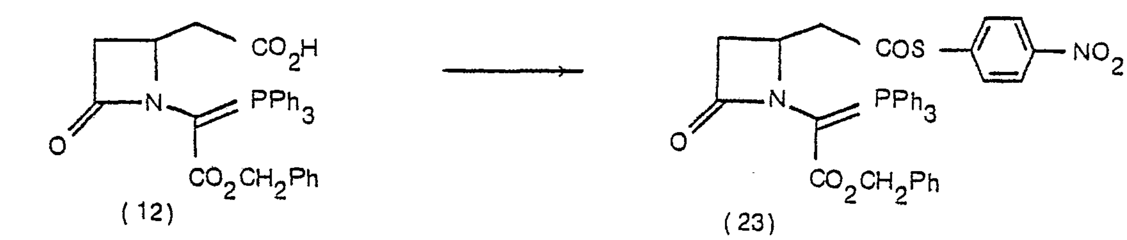

(12)

(23)

The acid (12, prepared as in Example 4a) (4.39g) in dry tetrahydrofuran (150ml), under argon, was treated with triethylamine (1.78ml) and diethylphosphorochloridate (2.13g). After stirring at room temperature for 3 hours, the reaction mixture was added to freshly prepared sodium *p*-

nitrophenylthiolate in tetrahydrofuran at 0° [prepared by treating dried *p*-nitrophenylthiol (1.4g) in dry tetrahydrofuran (75ml), under argon at 0° with sodium hydride (0.426g of a 50% suspension in oil)].

The stirred red mixture was kept below 10° (internal temperature) for 1.5 hours. After reducing the volume by evaporation, ethyl acetate (500ml) was added, and the reaction mixture was washed with brine, dried over magnesium sulphate and filtered, and the filtrate was evaporated. Chromatography of the residue on silica 60 (<230 mesh) 63 $\mu$m (80g), eluting with ethyl acetate gave the required thioester (25) (2.6g). Crystallisation from ethyl acetate/ether gave yellow crystals m.p. 169—70°C $\nu_{max}$ ($CHCl_3$) 1745, 1720 (sh), 1630 $cm^{-1}$. (*Found:* C, 67.53; H, 4.57; N, 3.93%. $C_{38}H_{31}N_2O_6SP$ requires C, 67.66; H, 4.60; N, 4.15%.

(b) *Preparation of Benzyl 3-p-nitrophenylthio-7-oxo-1-azabicyclo[3.2.0]hept-2-ene-2-carboxylate*

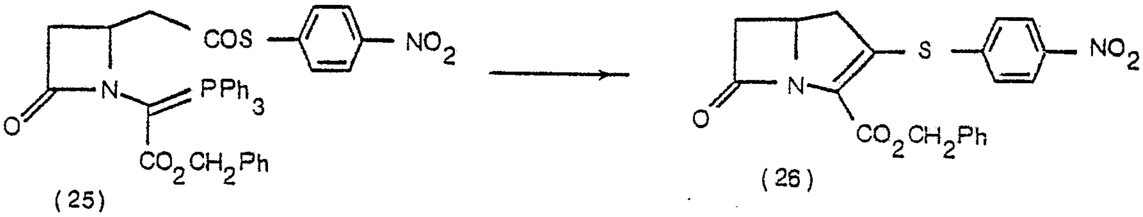

(25) (26)

The phosphorane (25) (1.0g) in dry toluene (600ml) was heated under an atmosphere of argon under reflux using a Dean-Stark apparatus to remove water. After 1.5 hours the dark solution was rapidly cooled, evaporated almost to dryness, then chromatographed on 30g silica 60 (230—400 mesh). Elution with 30% ethyl acetate in petroleum ether (60°—80°C) gave the required product (26), (41mg) as yellow crystals from ethyl acetate m.p. 126—31°. $\nu_{max}$ ($CHCl_3$) 1795, 1710, 1525, 1345$cm^{-1}$. $\delta$ *ppm* [$(CD_3)_2CO$] 3.00 (2H, d, J 10Hz, $C_4$—$CH_2$), 3.07 (1H, dd, J 17, 4Hz, C6—*H* trans), 3.52 (1H, dd, J 17, 6Hz, C6—*H* cis), 4.21 (1H, m, C5—*H*), 5.36 (2H, s, $CH_2$), 7.47 (5H, m, *Ph*), 7.92 and 8.32 (4H, ABq, J 9Hz, SP*h*$NO_2$). $\lambda_{max}$ (EtOH) 261 nm ($\varepsilon$ = 8,430), 312 nm ($\varepsilon$ = 10,219) 343 nm (sh) ($\varepsilon$ = 8,500).

## Example 11

## Sodium 7-oxo-3-phenylthio-1-azabicyclo[3.2.0]hept-2-ene-2-carboxylate

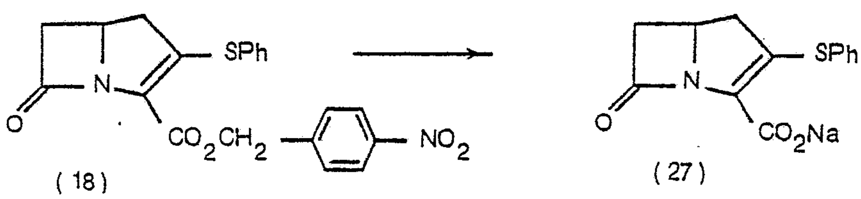

(18) (27)

The p-nitrobenzyl ester (18) (70mg) was dissolved in 30% aqueous dioxan containing 5% Pd/C (90mg) [prehydrogenated for twenty minutes]. The solution was hydrogenated at ambient temperature and pressure for one hour. Examination of the solution by u.v. showed a shift of chromophoric absorption of 316 nm and 266 nm for the p-nitrobenzyl ester to 314 nm and 251 nm respectively for the product. The solution was treated with one equivalent of $NaHCO_3$ (14.7mg) in water (2ml) and filtered through keiselguhr. The organic solvent was removed under reduced pressure until cloudiness occurred and extracted with ethyl acetate (3 x 10ml). The water was evaporated to low volume (approx. 2ml) and loaded onto a biogel P2 column. Elution with water and collection of 10ml fractions gave the sodium salt in fractions 11 and 12. The solvent was evaporated under high vacuum, and the solid obtained by evaporation of solvent twice from ethanol (2 x 10ml) and twice from toluene (2 x 15ml) to yield (27) as a light yellow solid (12mg). $\nu_{max}$ (KBr disc) 1755$cm^{-1}$, $\nu_{max}$ (EtOH) 302 nm.

Example 12

Phthalidyl 7-oxo-3-phenylthio-1-azabicyclo-[3.2.0]hept-2-ene-2-carboxylate

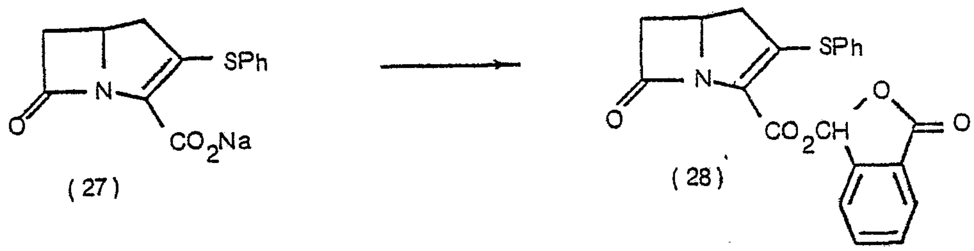

(27) (28)

The sodium slat (14mg) was dissolved in DMF (2ml) and treated with bromophthalide (10mg). The solution was stirred at RT for 3 hours, the solvent was evaporated, and the residue was dissolved in ethyl acetate and washed with brine (2 x 5ml). The organic phase was dried ($MgSO_4$) and evaporated to yield an oil which after chromatography gave (28) as an oil (3mg) $\nu_{max}$ ($CHCl_3$) 1795, 1730cm$^{-1}$. $\delta$ *ppm* ($CDCl_3$) 2.69 (2H, d, J9.5 Hz, C4—$CH_2$), 2.81 (1H, dd, J 16.5, 3.5Hz, C6—*H*a), 3.38 (1H, dd, J16.5, 5.5Hz, C6—*H*b) 4.07 (1H, m, C5—*H*) 6.55 ($\frac{1}{2}$H, s, $CO_2CH$ from one isomer) 7.20 to 8.00 (9$\frac{1}{2}$H, m, aromatics plus $CO_2CH$ from the second isomer) $\lambda_{max}$ 322 nm.

Example 13

Pivaloyloxymethyl 7-oxo-3-phenylthio-1-azabicyclo [3.2.0]hept-2-ene-2-carboxylate

(a) *Preparation of 1-(1'-pivaloyloxycarbonyl-1'-triphenylphosphoranylidenemethyl)-4-carboxymethyl-azetidin-2-one*

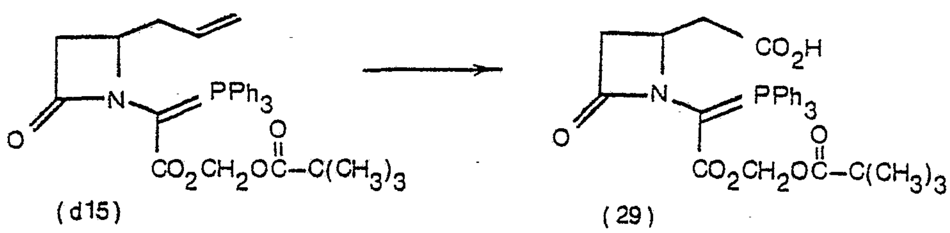

(d15) (29)

The phosphorane (d15, prepared as in Description 5) (258mg) was dissolved in dry methylene chloride (15ml) containing trifluoroacetic acid (0.8ml) and stirred at RT. for ten minutes. The solution was cooled to −70° and ozonised for 15 minutes until a blue colour persisted. Argon was passed through the solution to remove excess ozone and m-chloroperbenzoic acid (90mg) in methylene chloride (5ml) was added. The mixture was allowed to warm up to RT. and stirred overnight. The solvent was evaporated and the residue chromatographed (Kieselgel 60, <230 mesh 63 $\mu$m) to yield the phosphorane acid (29) as the trifluoroacetic acid salt. The salt was dissolved in methylene chloride and stirred with basic alumina (500mg) for one hour. The solution was filtered and evaporated to yield the phosphorane-acid (29) as a colourless foam (198 mg) $\nu_{max}$ ($CHCl_3$) 1730, 1600cm$^{-1}$.

(b) *Preparation of 1-(1'-pivaloyloxymethyloxycarbonyl-1'-triphenylphosphoranylidenemethyl)-4-(phenylthiocarbonylmethyl)azetidin-2-one.*

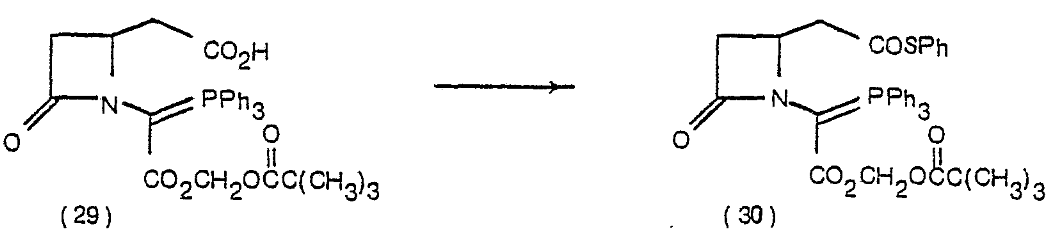

(29) (30)

The acid (29) (187mg) was dissolved in dry THF containing $Et_3N$ (34mg) and a solution of diethylphosphorochloridate (61mg) in dry THF (2ml) was added dropwise under argon. The reaction was stirred at RT. for three hours and thallium (I) phenylthiolate (105mg) was added. The reaction was stirred overnight, filtered and the solvent evaporated. The resulting oil was chromatographed on Merck Kieselgel 60 (<230 mesh 63$\mu$m) using ethyl acetate/petrol (60—80) as eluant to yield the title compound (30) as a colourless oil which crystallised from ether as a white solid m.p. 149—50° (120mg; 55%). $\nu_{max}$ ($CHCl_3$) 1735, 1700 (sh), 1630cm$^{-1}$.

(c) *Preparation of pivaloyloxymethyl 7-oxo-3-phenylthio-1-azabicyclo[3.2.0]hept-2-ene-2-carboxylate*

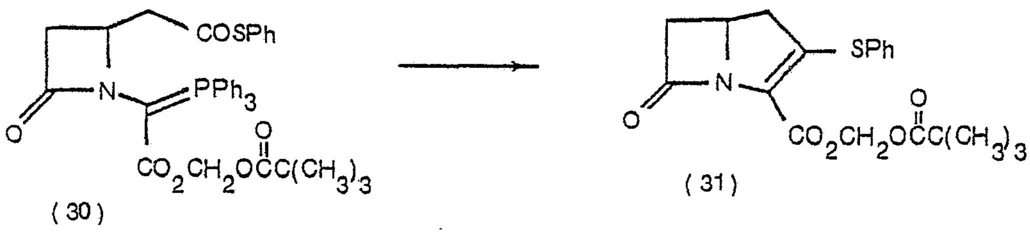

(30) (31)

The phosphorane (30) (218mg) was refluxed in dry toluene (250ml) under argon for 18 hours. The solvent was evaporated and the residue chromatographed on florisil (200—300 U.S. mesh) using slight pressure and ethyl acetate/petrol (60—80) as eluant. The title compound (31) was collected as a colourless oil (6mg; 5%) $\nu_{max}$ ($CHCl_3$) 1780, 1750, 1725cm$^{-1}$. $\delta$ *ppm* ($CDCl_3$), 1.22 (9H, s, $C(CH_3)_3$) 2.62 (2H, d, J 9.5Hz, C4—$CH_2$) 2.78 (1H, dd, J 16Hz, 3Hz, C6—*H*,trans), 3.36 (1H, dd, J 16Hz, 5Hz, C6—*H*, cis) 4.00 (1H, m, C5—*H*) 5.87 and 5.98 (2H, ABq, J 5.5Hz, $OCH_2O$) 7.40 (5H, m, *Ph*), $\lambda_{max}$ (EtOH) 319 nm.

Pharmacological Data

In Vitro Antibacterial Testing
Concentrations of Compounds
showing growth inhibition $\mu$g/ml

Dilution in DST agar
+ 5% Horse Blood

Inoculum $10^{-4}$ dilution

| ORGANISM | COMPOUND | | | | | | | | |
|---|---|---|---|---|---|---|---|---|---|
| | 10 | 14 | 18 | 20 | 22 | 24 | 26 | 27 | 28 |
| Bacillus subtilis A | <2 | 20 | 20 | 20 | 25 | ⩽2 | 10 | 1.9 | 5.5 |
| E.coli 0111 | >100 | 20 | 200 | 20 | >100 | >200 | >50 | 5.5 | 16.6 |
| Klebsiella aerogenes A | 20 | 200 | 20 | 200 | >100 | >200 | >50 | 5.5 | 5.5 |
| Proteus mirabilis C977 | >100 | 20 | 200 | 200 | >100 | >200 | >50 | 16.6 | 50 |
| Salmonella typhimurium CT10 | >100 | 20 | 200 | 200 | >100 | >200 | >50 | 16.6 | 50 |
| Serratia marcescens US20 | >100 | 20 | >200 | >200 | >100 | >200 | >50 | >50 | >50 |
| Shigella sonnei MB 11967 | >100 | 20 | 200 | 200 | >100 | >200 | >50 | 5.5 | 16.6 |
| Staph.aureus Oxford | >100 | 20 | 200 | 200 | 100 | ⩽2 | >50 | 50 | 16.6 |
| Staph.aureus Russell | 20 | 20 | 200 | 00 | >100 | 20 | 50 | 16.6 | 50 |
| Strep.pneumoniae | 20 | 20 | 2 | 20 | 10 | ⩽2 | 10 | — | — |
| Strep.pyogenes CN10 | 20 | 20 | 2 | 20 | 5 | ⩽2 | 10 | 5.5 | 5.5 |

*Note:* Compound (10) tested at 100, 20 and 2 $\mu$g/ml
Compounds (14), (18) and (20) tested at 200, 20 and 2 $\mu$g/ml
Compound (22) tested by twofold serial dilution
Compound (26) tested at 50, 10 and 1 $\mu$g/ml
Compounds (27) and (28) tested by 1 in 3 serial dilutions

**Claims**

1. A compound of the formula (II):

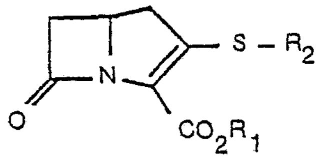

(II)

characterised in that $R_1$ is a group such that $CO_2R_1$ is a carboxylic acid group or a salt or ester thereof and $R_2$ is a phenyl group or a phenyl group substituted by one to four groups selected from $C_{1-4}$ alkyl, fluorine, chlorine, bromine, CN, $NO_2$, $COR_3$, $OR_3$, $SR_3$, $NH_2$, $NHCOR_3$, $NHCO_2R_3$, $CO_2R_3$ or $CO_2R_{10}$ where $R_3$ is $C_{1-4}$ alkyl, benzyl, fluorobenzyl, chlorobenzyl, bromobenzyl or nitrobenzyl and $CO_2R_{10}$ is carboxyl or a salt thereof, not more than three such groups being selected from fluorine, chlorine, bromine, CN, $NO_2$, $NH_2$, $COR_3$ or $CO_2R_3$ and not more than two such groups being selected from CN, $NO_2$ or $NH_2$.

2. A compound according to claim 1, characterised in that $R_2$ is a phenyl group or a phenyl group substituted by one or two groups selected from fluorine, chlorine, bromine, CN, $NO_2$, $COR_3$, $OR_3$, $NHCOR_3$, $NHCO_2R_3$ or $CO_2R_3$ where $R_3$ is a $C_{1-4}$ alkyl or benzyl group.

3. A compound according to claim 2, characterised in that $R_1$ is a group such that $CO_2R_1$ is an ester group.

4. A compound according to any one of claims 1, 2 or 3 characterised in that $R_2$ is phenyl, *p*-nitrophenyl, *p*-aminophenyl, *p*-acetamidophenyl or *p*-(4'-nitrobenzyloxycarbonylamino)phenyl.

5. A compound according to any one of claims 1, 2, 3, or 4 characterised in that $R_1$ is phthalidyl.

6. A compound according to any one of claims 1, 2, 3 or 4 characterised in that $R_1$ is *p*-nitrobenzyl.

7. A compound according to any one of claims 1, 2 or 4 characterised in that $R_1$ is a group such that the compound is a carboxylic acid salt.

8. A compound according to claim 7 characterised in that $R_1$ is sodium or potassium.

9. A compound according to claim 1, which compound is selected from t-butyl 7-oxo-3-*p*-nitrophenylthio-1-azabicyclo[3.2.0]hept-2-ene-2-carboxylate and benzyl 7-oxo-3-*p*-nitrophenylthio-1-azabicyclo[3.2.0]hept-2-ene-2-carboxylate.

10. A compound according to claim 1, which compound is selected from t-butyl 7-oxo-3-phenylthio-1-azabicyclo[3.2.0]hept-2-ene-2-carboxylate, methyl 7-oxo-3-phenylthio-1-azabicyclo[3.2.0]-hept-2-ene-2-carboxylate, benzyl 7-oxo-3-phenylthio-1-azabicyclo[3.2.0]hept-2-ene-2-carboxylate, *p*-nitrobenzyl 7-oxo-3-phenylthio-1-azabicyclo[3.2.0]hept-2-ene-2-carboxylate, pivaloyloxymethyl 7-oxo-3-phenylthio-1-azabicyclo [3.2.0]hept-2-ene-2-carboxylate, phthalidyl 7-oxo-3-phenylthio-1-azabicyclo[3.2.0]hept-2-ene-2-carboxylate, and sodium 7-oxo-3-phenylthio-1-azabicyclo[3.2.0]hept-2-ene-2-carboxylate.

11. A compound according to claim 1, which compound is selected from: benzyl 7-oxo-3-*p*-aminophenylthio-1-azabicyclo[3.2.0]hept-2-ene-2-carboxylate and benzyl 7-oxo-3-(4-*p*-nitrobenzyloxycarbonylamino)phenylthio-1-azabicyclo[3.2.0]hept-2-ene-2-carboxylate.

12. A compound according to claim 1, which compound is benzyl 7-oxo-3-*p*-acetamidophenylthio-1-azabicyclo[3.2.0]hept-2-ene-2-carboxylate.

13. A pharmaceutical composition characterised in that it comprises a compound according to any one of claims 1—12 and a pharmaceutically acceptable carrier.

14. A process for the preparation of a compound according to claim 1, characterised in that it comprises

a) the ring closing elimination of the elements of $O{=}PR_4R_5R_6$ from a compound of the formula (VII):

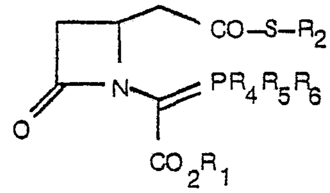

(VII)

wherein $CO_2R_1$ is an ester group, as defined in claim 1 and $R_4$, $R_5$ and $R_6$ are each $C_{1-4}$ alkyl, phenyl or di$C_{1-4}$alkylamino groups,

b) thereafter isolating the ester of the formula (II) according to claim 1 so formed,

c) where desired de-esterifying the ester to form a free acid or its salt,

d) and thereafter optionally salifying or esterifying the free carboxylic acid so formed, or

e) thereafter optionally converting the salt so formed into a free carboxylic acid, an ester or another salt.

15. A process for the preparation of a compound according to claim 3 characterised in that it comprises the ring closing elimination of the elements of $O{=}PR_4R_5R_6$ from a compound of the formula (VII):

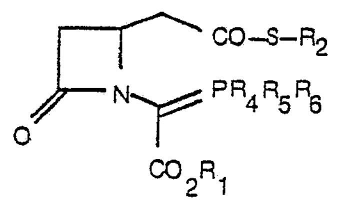

(VII)

wherein $CO_2R_1$ is an ester group, as defined in claim 3, and $R_4$, $R_5$ and $R_6$ are each lower alkyl, phenyl or diloweralkylamino groups.

16. A compound as claimed in claim 1 for use in the treatment of bacterial infection.

17. A composition as claimed in claim 13 which also comprises a penicillin or cephalosporin.

**Revendications**

1. Composé de formule (II):

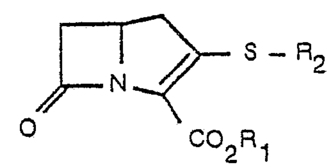

(II)

caractérisé en ce que $R_1$ est un groupe tel que $CO_2R_1$ soit un groupe acide carboxylique ou un sel ou ester de celui-ci et $R_2$ est un groupe phényle ou un groupe phényle substitué par un à quatre groupes choisis parmi les groupes alcoyle en $C_{1-4}$, fluor, chlore, brome, CN, $NO_2$, $COR_3$, $OR_3$, $SR_3$, $NH_2$, $NHCOR_3$, $NHCO_2R_3$, $CO_2R_3$ ou $CO_2R_{10}$, où $R_3$ est un groupe alcoyle en $C_{1-4}$, benzyle, fluorobenzyle, chlorobenzyle, bromobenzyle, ou nitrobenzyle, et $CO_2R_{10}$ est un carboxy ou un sel de celui-ci, un nombre ne dépassant pas trois de ces groupes étant choisis parmi le fluor, le chlore, le brome, CN, $NO_2$, $COR_3$ ou $CO_2R_3$, et un nombre ne dépassant pas deux de ces groupes étant choisis parmi CN, $NO_2$ ou $NH_2$.

2. Composé suivant la revendication 1, caractérisé en ce que $R_2$ est un groupe phényle ou bien un groupe phényle substitué par un ou deux groupes choisis parmi les substituants fluor, chlore, brome, CN, $NO_2$, $COR_3$, $OR_3$, $NHCOR_3$, $NHCO_2R_3$ ou $CO_2R_3$, où $R_3$ est un groupe alcoyle en $C_{1-4}$ ou benzyle.

3. Composé suivant la revendication 2, caractérisé en ce que $R_1$ est un groupe tel que $CO_2R_1$ soit un groupe ester.

4. Composé suivant l'une quelconque des revendications 1, 2 ou 3, caractérisé en ce que $R_2$ est un groupe phényle, p-nitrophényle, p-aminophényle, p-acétamidophényle ou p-(4'-nitrobenzyloxy-carbonylamino)phényle.

5. Composé suivant l'une quelconque des revendications 1, 2, 3 ou 4, caractérisé en ce que $R_1$ est un groũp phtalidyle.

6. Composé suivant l'une quelconque des revendications 1, 2, 3 ou 4, caractérisé en ce que $R_1$ est un groupe phtalidyle.

7. Composé suivant l'une quelconque des revendications 1, 2 ou 4, caractérisé en ce que $R_1$ est un groupe tel que le composé soit un sel d'acide carboxylique.

8. Composé suivant la revendication 7, caractérisé en ce que $R_1$ est du sodium ou du potassium.

9. Composé suivant la revendication 1, ce composé étant choisi parmi le 7-oxo-3-p-nitro-phénylthio-1-azabicyclo[3.2.0]hept-2-ène-carboxylate de t-butyle et le 7-oxo-3-p-nitro-hénylthio-1-azabicyclo[3.2.0]hept-2-ène-2-carboxylate de benzyle.

10. Composé suivant la revendication 1, ce composé étant choisi parmi le 7-oxo-3-phényl-thio-1-azabicyclo[3.2.0]hept-2-ène-2-carboxylate de t-butyle, le 7-oxo-3-phénylthio-1-azabi-cyclo[3.2.0]hept-2-ène-2-carboxylate de méthyle, le 7-oxo-3-phénylthio-1-azabicyclo[3.2.0]hept-2-ène-2-carboxylate de benzyle, le 7-oxo-3-phénylthio-1-azabicyclo[3.2.0]hept-2-ène-2-carboxy-

late de p-nitrobenzyle, le 7-oxo-3-phénylthio-1-azabicyclo[3.2.0]hept-2-ène-2-carboxylate de pivaloyloxyméthyle, le 7-oxo-3-phénylthio-1-azabicyclo[3.2.0]hept-2-ène-2-carboxylate de phtalidyle et le 7-oxo-3-phénylthio-1-azabicyclo[3.2.0]hept-2-ène-2-carboxylate de sodium.

11. Composé suivant la revendication 1, ce composé étant choisi parmi le 7-oxo-3-p-aminophénylthio-1-azabicyclo[3.2.0]hept-2-ène-2-carboxylate de benzyle et le 7-oxo-3-(4-p-nitrobenzyloxycarbonylamino)phénylthio-1-azabicyclo[3.2.0]hept-2-ène-2-carboxylate de benzyle.

12. Composé suivant la revendication 1, ce composé étant le 7-oxo-3-p-acétamidophénylthio-1-azabicyclo[3.2.0]hept-2-ène-2-carboxylate de benzyle.

13. Composition pharmaceutique, caractérisée en ce qu'elle renferme un composé suivant l'une quelconque des revendications 1 à 12 et un véhicule ou excipient pharmaceutiquement acceptable.

14. Procédé pour la préparation d'un composé suivant la revendication 1, caractérisé en ce qu'il consiste:

(a) à effectuer l'élimination par fermeture de cycle des éléments de $O{=}PR_4R_5R_6$ à partir d'un composé de formule (VII):

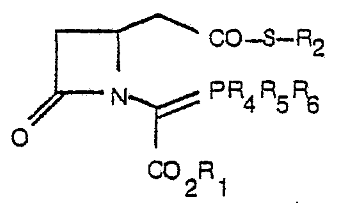

(VII)

dans laquelle $CO_2R_1$ est un groupe ester, tel que défini dans la revendication 1, et $R_4$, $R_5$ et $R_6$ sont chacun un groupe alcoyle en $C_{1-4}$, phényle ou dialcoyl $(C_{1-4})$ amino.

(b) à isoler ensuite l'ester de formule (II) suivant la revendication 1 ainsi formé;

(c) lorsque cela est désirable à désestérifier l'ester pour former un acide libre ou son sel;

(d) et éventuellement à salifier ou estérifier ensuite l'acide carboxylique libre ainsi formé; ou

(e) éventuellement à convertir ensuite le sel ainsi formé en un acide carboxylique libre, un ester ou un autre sel.

15. Procédé pour la préparation d'un composé suivant la revendication 3, caractérisé en ce qu'on effectue l'élimination par fermeture du cycle des éléments de $O = PR_4R_5R_6$ à partir d'un composé de formule (VII):

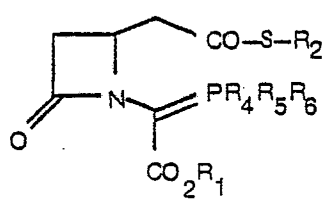

(VII)

dans laquelle $CO_2R_1$ est un groupe ester tel que défini dans la revendication 3, et $R_4$, $R_5$ et $R_6$ sont chacun un groupe alcoyle inférieur, phényle ou dialcoyl (inférieur) amino.

16. Composé suivant la revendication 1, destiné à être utilisé pour le traitement des infections bactériennes.

17. Composition suivant la revendication 13, renfermant également une pénicilline ou une céphalosporine.

**Patentansprüche**

1. Eine Verbindung der Formel (II):

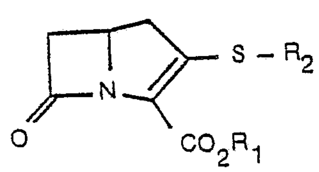

(II)

dadurch gekennzeichnet, daß $R_1$ eine solche Gruppe ist, daß $CO_2R_1$ eine Carbonsäuregruppe oder ein Salz oder Ester derselben ist und $R_2$ eine Phenylgruppe oder eine Phenylgruppe ist, die mit einer bis 4

Gruppe(n) substituiert ist, ausgewählt aus $C_{1-4}$-Alkyl, Fluor, Chlor, Brom, CN, $NO_2$, $COR_3$, $OR_3$, $SR_3$, $NH_2$, $NHCOR_3$, $NHCO_2R_3$, $CO_2R_3$ oder $CO_2R_{10}$, wobei $R_3$ eine $C_{1-4}$-Alkyl-, Benzyl-, Fluorbenzyl-, Chlorbenzyl-, Brombenzyl- oder Nitrobenzylgruppe ist und $CO_2R_{10}$ eine Carboxylgruppe oder ein Salz derselben ist, wobei nicht mehr als 3 solche Gruppen aus Fluor, Chlor, Brom, CN, $NO_2$, $NH_2$, $COR_3$ oder $CO_2R_3$ ausgewählt sind und nicht mehr als 2 solche Gruppen aus CN, $NO_2$ oder $NH_2$ ausgewählt sind.

2. Eine Verbindung gemäß Anspruch 1, dadurch gekennzeichnet, daß $R_2$ eine Phenylgruppe oder eine Phenylgruppe ist, die mit einer oder zwei Gruppe(n) substituiert ist, ausgewählt aus Fluor, Chlor, Brom, CN, $NO_2$, $COR_3$, $OR_3$, $NHCOR_3$, $NHCO_2R_3$ oder $CO_2R_3$, wobei $R_3$ eine niedere $C_{1-4}$-Alkyl- oder Benzylgruppe ist.

3. Eine Verbindung gemäß Anspruch 2, dadurch gekennzeichnet, daß $R_1$ eine solche Gruppe ist, daß $CO_2R_1$ eine Estergruppe ist.

4. Eine Verbindung gemäß einem der Ansprüche 1, 2 oder 3, dadurch gekennzeichnet, daß $R_2$ eine Phenyl-, p-Nitrophenyl-, p-Aminophenyl-, p-Acetamidophenyl- oder p-(4'-Nitrobenzyloxycarbonylamino)-phenylgruppe ist.

5. Eine Verbindung gemäß einem der Ansprüche 1, 2, 3 oder 4, dadurch gekennzeichnet, daß $R_1$ eine Phtalidylgruppe ist.

6. Eine Verbindung gemäß einem der Ansprüche 1, 2, 3 oder 4, dadurch gekennzeichnet, daß $R_1$ eine p-Nitrobenzylgruppe ist.

7. Eine Verbindung gemäß einem der Ansprüche 1, 2 oder 4, dadurch gekennzeichnet, daß $R_1$ eine solche Gruppe ist, daß die Verbindung ein Carbonsäuresalz ist.

8. Eine Verbindung gemäß Anspruch 7, dadurch gekennzeichnet, daß $R_1$ Natrium oder Kalium ist.

9. Eine Verbindung gemäß Anspruch 1, wobei die Verbindung aus 7-oxo-3-p-nitrophenylthio-1-azabicyclo[3.2.0]hept-2-en-2-carbonsäure-tert.-butylester und 7-Oxo-3-p-nitrophenylthio-1-azabicyclo[3.2.0] hept-2-en-2-carbonsäurebenzylester ausgewählt ist.

10. Eine Verbindung gemäß Anspruch 1, wobei die Verbindung aus 7-Oxo-3-phenylthio-1-azabicyclo[3.2.0]hept-2-en-2-carbonsäure-tert.-butylester, 7-Oxo-3-phenylthio-1-azabicyclo[3.2.0]hept-2-en-2-carbonsäure-methylester, 7-Oxo-3-phenylthio-1-azabicyclo[3.2.0]hept-2-en-2-carbonsäure-benzylester, 7-Oxo-3-phenylthio-1-azbicyclo[3.2.0]hept-2-en-2-carbonsäure-p-nitrobenzylester, 7-Oxo-3-phenylthio-1-azabicyclo[3.2.0]hept-2-en-2-carbonsäure-pivaloyloxymethylester, 7-Oxo-3-phenylthio-1-azabicyclo[3.2.0]hept-2-en-2-carbonsäurephthalidylester und 7-Oxo-3-phenylthio-1-azabicyclo[3.2.0]hept-2-en-2-carbonsäure-natriumsalz ausgewählt ist.

11. Eine Verbindung gemäß Anspruch 1, wobei die Verbindung ausgewählt ist aus: 7-Oxo-3-p-aminophenylthio-1-azabicyclo[3.2.0]hept-2-en-2-carbonsäure-benzylester und 7-Oxo-3-(4-p-nitrobenzyloxycarbonylamino)-phenylthio-1-azabicyclo[3.2.0]hept-2-en-2-carbonsäure-benzylester.

12. Eine Verbindung gemäß Anspruch 1, wobei die Verbindung 7-Oxo-3-p-acetamidophenylthio-1-azabicyclo[3.2.0]hept-2-en-2-carbonsäure-benzylester ist.

13. Eine pharmazeutische Zusammensetzung, dadurch gekennzeichnet, daß sie eine Verbindung gemäß einem der Ansprüche 1 bis 12 und einen pharmakologisch verträglichen Träger enthält.

14. Ein Verfahren zur Herstellung einer Verbindung gemäß Anspruch 1, dadurch gekennzeichnet, daß es die Maßnahmen umfaßt, daß

a) die Elemente $O{=}PR_4R_5R_6$ aus einer Verbindung der Formel (VII) durch Ringschluß eliminiert werden:

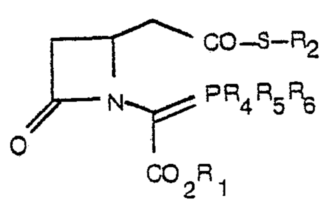

(VII)

wobei $CO_2R_1$ eine Estergruppe ist, wie in Anspruch 1 definiert, und $R_4$, $R_5$ und $R_6$ je eine $C_{1-4}$-Alkyl-, Phenyl- oder Di-$C_{1-4}$-alkylaminogruppe ist,

b) anschließend der so gebildete Ester der Formel (II) gemäß Anspruch 1 isoliert wird,

c) wo es gewünscht wird, der Ester zu einer freien Säure oder deren Salz verseift wird,

d) und anschließend gegebenenfalls die so gebildete freie Säure in ein Salz oder einen Ester umgewandelt wird, oder

e) anschließend gegebenenfalls das so gebildete Salz in eine freie Säure, einen Ester oder ein anderes Salz umgewandelt wird.

15. Ein Verfahren zur Herstellung einer Verbindung gemäß Anspruch 3, dadurch gekennzeichnet, daß es die Maßnahmen umfaßt, daß die Elemente $O{=}PR_4R_5R_6$ aus einer Verbindung der Formel (VII) durch Ringschluß eliminiert werden:

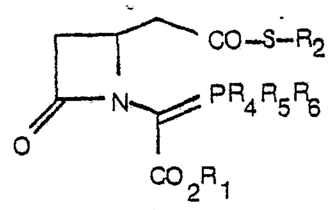

(VII)

wobei $CO_2R_1$ eine Estergruppe ist, wie in Anspruch 3 definiert, und $R_4$, $R_5$ und $R_6$ je eine niedere Alkyl-, Phenyl- oder Di-nieder-alkylaminogruppe ist.

16. Eine Verbindung wie in Anspruch 1 beansprucht, zur Verwendung in der Behandlung von bakteriellen Infektionen.

17. Eine Zusammensetzung wie in Anspruch 13 beansprucht, die auch ein Penicillin oder Cephalosphorin enthält.